(19) Europäisches Patentamt
European Patent Office
Office européen des brevets

(11) EP 2 695 949 B1

(12) **EUROPEAN PATENT SPECIFICATION**

(45) Date of publication and mention of the grant of the patent:
**19.10.2016 Bulletin 2016/42**

(51) Int Cl.:
***C12Q 1/68*** *(2006.01)*

(21) Application number: **12180126.0**

(22) Date of filing: **10.08.2012**

(54) **Nucleic acid based nanopores or transmembrane channels and their uses**

Nukleinsäurebasierte Nanoporen oder Transmembran-Kanäle und ihre Verwendungen

Nanopores à base d'acide nucléique ou canaux transmembranaires et leurs utilisations

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.02.2014 Bulletin 2014/07**

(73) Proprietor: **Technische Universität München
80333 München (DE)**

(72) Inventors:
- **Langecker, Martin
80939 München (DE)**
- **Arnaut, Vera
85748 Garching (DE)**
- **Martin, Thomas
83115 Neubeuern (DE)**
- **Simmel, Friedrich C.
80336 München (DE)**
- **Dietz, Hendrik
80804 München (DE)**

(74) Representative: **Engelhard, Markus
Boehmert & Boehmert
Anwaltspartnerschaft mbB
Patentanwälte Rechtsanwälte
Pettenkoferstrasse 20-22
80336 München (DE)**

(56) References cited:
- **NICHOLAS A. W. BELL ET AL: "DNA Origami Nanopores", NANO LETTERS, vol. 12, no. 1, 11 January 2012 (2012-01-11), pages 512-517, XP055048370, ISSN: 1530-6984, DOI: 10.1021/nl204098n**
- **RUOSHAN WEI ET AL: "DNA Origami Gatekeepers for Solid-State Nanopores", ANGEWANDTE CHEMIE INTERNATIONAL EDITION, vol. 51, no. 20, 14 May 2012 (2012-05-14), pages 4864-4867, XP055048404, ISSN: 1433-7851, DOI: 10.1002/anie.201200688**
- **MARK S. KAUCHER ET AL: "A Unimolecular G-Quadruplex that Functions as a Synthetic Transmembrane Na + Transporter", JOURNAL OF THE AMERICAN CHEMICAL SOCIETY, vol. 128, no. 1, 1 January 2006 (2006-01-01), pages 38-39, XP055048352, ISSN: 0002-7863, DOI: 10.1021/ja056888e**
- **PARISA AKHSHI ET AL: "Free-Energy Landscapes of Ion Movement through a G-Quadruplex DNA Channel", ANGEWANDTE CHEMIE INTERNATIONAL EDITION, vol. 51, no. 12, 19 March 2012 (2012-03-19), pages 2850-2854, XP055048320, ISSN: 1433-7851, DOI: 10.1002/anie.201107700**
- **SCOTT L. FORMAN ET AL: "Toward Artificial Ion Channels: A Lipophilic G-Quadruplex", JOURNAL OF THE AMERICAN CHEMICAL SOCIETY, vol. 122, no. 17, 1 May 2000 (2000-05-01), pages 4060-4067, XP055048357, ISSN: 0002-7863, DOI: 10.1021/ja9925148**
- **M. LANGECKER ET AL: "Synthetic Lipid Membrane Channels Formed by Designed DNA Nanostructures", SCIENCE, vol. 338, no. 6109, 16 November 2012 (2012-11-16), pages 932-936, XP055048403, ISSN: 0036-8075, DOI: 10.1126/science.1225624**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Technische Universität Munchen

<120> Nucleic acid based nanopores or transmembrane channels and their uses

<130> U30443EP

<160> 139

<170> PatentIn version 3.5

<210> 1
<211> 7249
<212> DNA
<213> Artificial Sequence

<220>

<223> m13mp18 Scaffold sequence

<400> 1

```
aatgctacta ctattagtag aattgatgcc accttttcag ctcgcgcccc aaatgaaaat      60
atagctaaac aggttattga ccatttgcga aatgtatcta atggtcaaac taaatctact     120
cgttcgcaga attgggaatc aactgttata tggaatgaaa cttccagaca ccgtacttta     180
gttgcatatt taaaacatgt tgagctacag cattatattc agcaattaag ctctaagcca     240
tccgcaaaaa tgacctctta tcaaaaggag caattaaagg tactctctaa tcctgacctg     300
ttggagtttg cttccggtct ggttcgcttt gaagctcgaa ttaaaacgcg atatttgaag     360
tctttcgggc ttcctcttaa tctttttgat gcaatccgct ttgcttctga ctataatagt     420
cagggtaaag acctgatttt tgatttatgg tcattctcgt tttctgaact gtttaaagca     480
tttgaggggg attcaatgaa tatttatgac gattccgcag tattggacgc tatccagtct     540
aaacatttta ctattacccc ctctggcaaa acttcttttg caaaagcctc tcgctatttt     600
ggtttttatc gtcgtctggt aaacgagggt tatgatagtg ttgctcttac tatgcctcgt     660
aattcctttt ggcgttatgt atctgcatta gttgaatgtg gtattcctaa atctcaactg     720
atgaatcttt ctacctgtaa taatgttgtt ccgttagttc gttttattaa cgtagatttt     780
tcttcccaac gtcctgactg gtataatgag ccagttctta aaatcgcata aggtaattca     840
caatgattaa agttgaaatt aaaccatctc aagcccaatt tactactcgt tctggtgttt     900
ctcgtcaggg caagccttat tcactgaatg agcagctttg ttacgttgat ttgggtaatg     960
aatatccggt tcttgtcaag attactcttg atgaaggtca gccagcctat gcgcctggtc    1020
tgtacaccgt tcatctgtcc tctttcaaag ttggtcagtt cggttccctt atgattgacc    1080
gtctgcgcct cgttccggct aagtaacatg gagcaggtcg cggatttcga cacaatttat    1140
caggcgatga tacaaatctc cgttgtactt tgtttcgcgc ttggtataat cgctgggggt    1200
caaagatgag tgttttagtg tattcttttg cctctttcgt tttaggttgg tgccttcgta    1260
gtggcattac gtattttacc cgtttaatgg aaacttcctc atgaaaaagt ctttagtcct    1320
```

caaagcctct gtagccgttg ctaccctcgt tccgatgctg tctttcgctg ctgagggtga 1380

cgatcccgca aaagcggcct ttaactccct gcaagcctca gcgaccgaat atatcggtta 1440

tgcgtgggcg atggttgttg tcattgtcgg cgcaactatc ggtatcaagc tgtttaagaa 1500

attcacctcg aaagcaagct gataaaccga tacaattaaa ggctcctttt ggagcctttt 1560

ttttggagat tttcaacgtg aaaaaattat tattcgcaat tcctttagtt gttcctttct 1620

attctcactc cgctgaaact gttgaaagtt gtttagcaaa atcccataca gaaaattcat 1680

ttactaacgt ctggaaagac gacaaaactt tagatcgtta cgctaactat gagggctgtc 1740

tgtggaatgc tacaggcgtt gtagtttgta ctggtgacga aactcagtgt tacggtacat 1800

gggttcctat tgggcttgct atccctgaaa atgagggtgg tggctctgag ggtggcggtt 1860

ctgagggtgg cggttctgag ggtggcggta ctaaacctcc tgagtacggt gatacaccta 1920

ttccgggcta tacttatatc aaccctctcg acggcactta tccgcctggt actgagcaaa 1980

accccgctaa tcctaatcct tctcttgagg agtctcagcc tcttaatact ttcatgtttc 2040

agaataatag gttccgaaat aggcaggggg cattaactgt ttatacgggc actgttactc 2100

aaggcactga ccccgttaaa acttattacc agtacactcc tgtatcatca aaagccatgt 2160

atgacgctta ctggaacggt aaattcagag actgcgcttt ccattctggc tttaatgagg 2220

atttatttgt ttgtgaatat caaggccaat cgtctgacct gcctcaacct cctgtcaatg 2280

ctggcggcgg ctctggtggt ggttctggtg gcggctctga gggtggtggc tctgagggtg 2340

gcggttctga gggtggcggc tctgagggag gcggttccgg tggtggctct ggttccggtg 2400

attttgatta tgaaaagatg gcaaacgcta ataagggggc tatgaccgaa aatgccgatg 2460

aaaacgcgct acagtctgac gctaaaggca aacttgattc tgtcgctact gattacggtg 2520

ctgctatcga tggtttcatt ggtgacgttt ccggccttgc taatggtaat ggtgctactg 2580

gtgattttgc tggctctaat tcccaaatgg ctcaagtcgg tgacggtgat aattcacctt 2640

taatgaataa tttccgtcaa tatttacctt ccctccctca atcggttgaa tgtcgccctt 2700

ttgtctttgg cgctggtaaa ccatatgaat tttctattga ttgtgacaaa ataaacttat 2760

tccgtggtgt ctttgcgttt cttttatatg ttgccacctt tatgtatgta ttttctacgt 2820

ttgctaacat actgcgtaat aaggagtctt aatcatgcca gttcttttgg gtattccgtt 2880

attattgcgt ttcctcggtt tccttctggt aactttgttc ggctatctgc ttacttttct 2940

taaaaagggc ttcggtaaga tagctattgc tatttcattg tttcttgctc ttattattgg 3000

gcttaactca attcttgtgg gttatctctc tgatattagc gctcaattac cctctgactt 3060

tgttcagggt gttcagttaa ttctcccgtc taatgcgctt ccctgttttt atgttattct 3120

ctctgtaaag gctgctattt tcatttttga cgttaaacaa aaaatcgttt cttatttgga 3180

ttgggataaa taatatggct gtttattttg taactggcaa attaggctct ggaaagacgc 3240

```
tcgttagcgt tggtaagatt caggataaaa ttgtagctgg gtgcaaaata gcaactaatc     3300
ttgatttaag gcttcaaaac ctcccgcaag tcgggaggtt cgctaaaacg cctcgcgttc     3360
ttagaatacc ggataagcct tctatatctg atttgcttgc tattgggcgc ggtaatgatt     3420
cctacgatga aaataaaaac ggcttgcttg ttctcgatga gtgcggtact tggtttaata     3480
cccgttcttg gaatgataag gaaagacagc cgattattga ttggtttcta catgctcgta     3540
aattaggatg ggatattatt tttcttgttc aggacttatc tattgttgat aaacaggcgc     3600
gttctgcatt agctgaacat gttgtttatt gtcgtcgtct ggacagaatt actttacctt     3660
ttgtcggtac tttatattct cttattactg gctcgaaaat gcctctgcct aaattacatg     3720
ttggcgttgt taaatatggc gattctcaat taagccctac tgttgagcgt tggctttata     3780
ctggtaagaa tttgtataac gcatatgata ctaaacaggc tttttctagt aattatgatt     3840
ccggtgttta ttcttattta acgccttatt tatcacacgg tcggtatttc aaaccattaa     3900
atttaggtca gaagatgaaa ttaactaaaa tatatttgaa aaagttttct cgcgttcttt     3960
gtcttgcgat tggatttgca tcagcattta catatagtta tataacccaa cctaagccgg     4020
aggttaaaaa ggtagtctct cagacctatg attttgataa attcactatt gactcttctc     4080
agcgtcttaa tctaagctat cgctatgttt tcaaggattc taagggaaaa ttaattaata     4140
gcgacgattt acagaagcaa ggttattcac tcacatatat tgatttatgt actgtttcca     4200
ttaaaaaagg taattcaaat gaaattgtta aatgtaatta attttgtttt cttgatgttt     4260
gtttcatcat cttcttttgc tcaggtaatt gaaatgaata attcgcctct gcgcgatttt     4320
gtaacttggt attcaaagca atcaggcgaa tccgttattg tttctcccga tgtaaaaggt     4380
actgttactg tatattcatc tgacgttaaa cctgaaaatc tacgcaattt ctttatttct     4440
gttttacgtg caaataattt tgatatggta ggttctaacc cttccattat tcagaagtat     4500
aatccaaaca atcaggatta tattgatgaa ttgccatcat ctgataatca ggaatatgat     4560
gataattccg ctccttctgg tggtttcttt gttccgcaaa atgataatgt tactcaaact     4620
tttaaaatta ataacgttcg ggcaaaggat ttaatacgag ttgtcgaatt gtttgtaaag     4680
tctaatactt ctaaatcctc aaatgtatta tctattgacg gctctaatct attagttgtt     4740
agtgctccta aagatatttt agataacctt cctcaattcc tttcaactgt tgatttgcca     4800
actgaccaga tattgattga gggtttgata tttgaggttc agcaaggtga tgctttagat     4860
ttttcatttg ctgctggctc tcagcgtggc actgttgcag gcggtgttaa tactgaccgc     4920
ctcacctctg ttttatcttc tgctggtggt tcgttcggta tttttaatgg cgatgtttta     4980
gggctatcag ttcgcgcatt aaagactaat agccattcaa aaatattgtc tgtgccacgt     5040
attcttacgc tttcaggtca gaagggttct atctctgttg gccagaatgt cccttttatt     5100
actggtcgtg tgactggtga atctgccaat gtaaataatc catttcagac gattgagcgt     5160
caaaatgtag gtatttccat gagcgttttt cctgttgcaa tggctggcgg taatattgtt     5220
```

ctggatatta ccagcaaggc cgatagtttg agttcttcta ctcaggcaag tgatgttatt 5280

actaatcaaa gaagtattgc tacaacggtt aatttgcgtg atggacagac tcttttactc 5340

ggtggcctca ctgattataa aaacacttct caggattctg gcgtaccgtt cctgtctaaa 5400

atccctttaa tcggcctcct gtttagctcc cgctctgatt ctaacgagga aagcacgtta 5460

tacgtgctcg tcaaagcaac catagtacgc gccctgtagc ggcgcattaa gcgcggcggg 5520

tgtggtggtt acgcgcagcg tgaccgctac acttgccagc gccctagcgc ccgctccttt 5580

cgctttcttc ccttcctttc tcgccacgtt cgccggcttt ccccgtcaag ctctaaatcg 5640

ggggctccct ttagggttcc gatttagtgc tttacggcac ctcgacccca aaaaacttga 5700

tttgggtgat ggttcacgta gtgggccatc gccctgatag acggtttttc gccctttgac 5760

gttggagtcc acgttcttta atagtggact cttgttccaa actggaacaa cactcaaccc 5820

tatctcgggc tattcttttg atttataagg gattttgccg atttcggaac caccatcaaa 5880

caggattttc gcctgctggg gcaaaccagc gtggaccgct tgctgcaact ctctcagggc 5940

caggcggtga agggcaatca gctgttgccc gtctcactgg tgaaaagaaa aaccaccctg 6000

gcgcccaata cgcaaaccgc ctctccccgc gcgttggccg attcattaat gcagctggca 6060

cgacaggttt cccgactgga aagcgggcag tgagcgcaac gcaattaatg tgagttagct 6120

cactcattag gcaccccagg ctttacactt tatgcttccg gctcgtatgt tgtgtggaat 6180

tgtgagcgga taacaatttc acacaggaaa cagctatgac catgattacg aattcgagct 6240

cggtacccgg ggatcctcta gagtcgacct gcaggcatgc aagcttggca ctggccgtcg 6300

ttttacaacg tcgtgactgg gaaaaccctg gcgttaccca acttaatcgc cttgcagcac 6360

atcccccttt cgccagctgg cgtaatagcg aagaggcccg caccgatcgc ccttcccaac 6420

agttgcgcag cctgaatggc gaatggcgct ttgcctggtt tccggcacca gaagcggtgc 6480

cggaaagctg gctggagtgc gatcttcctg aggccgatac tgtcgtcgtc ccctcaaact 6540

ggcagatgca cggttacgat gcgcccatct acaccaacgt gacctatccc attacggtca 6600

atccgccgtt tgttcccacg gagaatccga cgggttgtta ctcgctcaca tttaatgttg 6660

atgaaagctg gctacaggaa ggccagacgc gaattatttt tgatggcgtt cctattggtt 6720

aaaaaatgag ctgatttaac aaaaatttaa tgcgaatttt aacaaaatat taacgtttac 6780

aatttaaata tttgcttata caatcttcct gtttttgggg cttttctgat tatcaaccgg 6840

ggtacatatg attgacatgc tagttttacg attaccgttc atcgattctc ttgtttgctc 6900

cagactctca ggcaatgacc tgatagcctt tgtagatctc tcaaaaatag ctaccctctc 6960

cggcattaat ttatcagcta gaacggttga atatcatatt gatggtgatt tgactgtctc 7020

cggcctttct cacccttttg aatctttacc tacacattac tcaggcattg catttaaaat 7080

atatgagggt tctaaaaatt tttatccttg cgttgaaata aaggcttctc ccgcaaaagt 7140

attacagggt cataatgttt ttggtacaac cgatttagct ttatgctctg aggctttatt 7200

gcttaatttt gctaattctt tgccttgcct gtatgattta ttggatgtt 7249

<210> 2
<211> 42
<212> DNA
<213> Artificial Sequence

<220>
<223> Staple oligonucleotide

<400> 2
gtcagttcct gcaataggaa ttgataatcg gctgtctttc ct 42

<210> 3
<211> 42
<212> DNA
<213> Artificial Sequence

<220>
<223> Staple oligonucleotide

<400> 3
agctattttt gagaactagc actaaaacaa ttgtgtcaat ca 42

<210> 4
<211> 49
<212> DNA
<213> Artificial Sequence

<220>
<223> Staple oligonuleotide

<400> 4
agtaccacca ctacgaatac atgtcaatcc aaaaacagga agattgtat 49

<210> 5
<211> 21
<212> DNA
<213> Artificial Sequence

<220>
<223> Staple oligonucleotide

<400> 5
tttcattgtc gctgcagctt g 21

<210> 6
<211> 42
<212> DNA
<213> Artificial Sequence

<220>
<223> Staple oligonucleotide

<400> 6

atttacgctg tttacagacg acgacaatca ttttcaaggt ga 42

<210> 7
<211> 42
<212> DNA
<213> Artificial Sequence

<220>
<223> Staple oligonucleotide

<400> 7
gaggctggcc tttaggccgg aatagaagaa caagccagca aa 42

<210> 8
<211> 35
<212> DNA
<213> Artificial Sequence

<220>
<223> Staple oligonucleotide

<400> 8
gtattaagcc gttccagtaa gcgtcatatc tgaaa 35

<210> 9
<211> 35
<212> DNA
<213> Artificial Sequence

<220>
<223> Staple oligonucleotide

<400> 9
gcctattggt catagccccc tcgcctccag ccgcc 35

<210> 10
<211> 35
<212> DNA
<213> Artificial Sequence

<220>
<223> Staple oligonucleotide

<400> 10
gccccctagt gtactaaatc ctcattaacc accag 35

<210> 11
<211> 42
<212> DNA
<213> Artificial Sequence

<220>
<223> Staple oligonucleotide

<400> 11
ataggtgacc gaactgacca aacaagaacc ggatactcca gc 42

<210> 12

<211> 42
<212> DNA
<213> Artificial Sequence

<220>
<223> Staple oligonucleotide

<400> 12
gtactcaagt ttcgttttaa gaaacgaaca acgcggtgag ac 42

<210> 13
<211> 42
<212> DNA
<213> Artificial Sequence

<220>
<223> Staple oligonucleotide

<400> 13
tcagaacaga cttagccgga atgctcattc agtgatattg ag 42

<210> 14
<211> 42
<212> DNA
<213> Artificial Sequence

<220>
<223> Staple oligonucleotide

<400> 14
aacccatacc agtcaggaca acagaataag gcttgcatac ga 42

<210> 15
<211> 42
<212> DNA
<213> Artificial Sequence

<220>
<223> Staple oligonucleotide

<400> 15
gtagcatgtt taccaaagga aggcaaaatt gccccagcag gc 42

<210> 16
<211> 42
<212> DNA
<213> Artificial Sequence

<220>
<223> Staple oligonucleotide

<400> 16
acgatctttg ctaagattag tgggaggtag ttgaaatatc tg 42

<210> 17
<211> 35
<212> DNA
<213> Artificial Sequence

<220>
<223> Staple oligonucleotide

<400> 17
tcagagcacc gccaccctca gactgtagca tgaaa 35

<210> 18
<211> 42
<212> DNA
<213> Artificial Sequence

<220>
<223> Staple oligonucleotide

<400> 18
gccagcaaaa caaatggtaa taagttttta taaacgtttg cc 42

<210> 19
<211> 42
<212> DNA
<213> Artificial Sequence

<220>
<223> Staple oligonucleotide

<400> 19
aaggaacgac ttcaaatatc gcaaatgccg tccaagggcg ct 42

<210> 20
<211> 42
<212> DNA
<213> Artificial Sequence

<220>
<223> Staple oligonucleotide

<400> 20
tgggattaaa gtttagaata aattaactaa tatactaacg ga 42

<210> 21
<211> 21
<212> DNA
<213> Artificial Sequence

<220>
<223> Staple oligonucleotide

<400> 21
atgaattcag ctacttagcg a 21

<210> 22
<211> 28
<212> DNA
<213> Artificial Sequence

<220>
<223> Staple oligonucleotide

<400> 22
ctcagagccg ccaccatttt ctcggaac 28

<210> 23
<211> 28
<212> DNA
<213> Artificial Sequence

<220>
<223> Staple oligonucleotide

<400> 23
gccaccaccg gaactattag cagttaat 28

<210> 24
<211> 48
<212> DNA
<213> Artificial Sequence

<220>
<223> Staple oligonucleotide

<400> 24
aaaaaggctc ggaattgcga ataaagatta agaggaagaa aacgagaa 48

<210> 25
<211> 42
<212> DNA
<213> Artificial Sequence

<220>
<223> Staple oligonucleotide

<400> 25
atcagcttgc ttactcaaca gtcatagtag caacactatc at 42

<210> 26
<211> 42
<212> DNA
<213> Artificial Sequence

<220>
<223> Staple oligonucleotide

<400> 26
caagtttaga ctccaagggc gcacaatcaa ttcttcataa tt 42

<210> 27
<211> 42
<212> DNA
<213> Artificial Sequence

<220>
<223> Staple oligonucleotide

<400> 27
gcgtcagaga gtaaacgttc caccacccct ctgaatttac ca 42

<210> 28
<211> 42
<212> DNA
<213> Artificial Sequence

<220>
<223> Staple oligonucleotide

<400> 28
gatagcacca ccgacttgag cagaaaccaa tcagtgccag ct 42

<210> 29
<211> 42
<212> DNA
<213> Artificial Sequence

<220>
<223> Staple oligonucleotide

<400> 29
attatcaccg tcgcatttta ggcagaggaa acaacatgtt aa 42

<210> 30
<211> 42
<212> DNA
<213> Artificial Sequence

<220>
<223> Staple oligonucleotide

<400> 30
cgccagcagg cggtgctcaa cccattaggc tatattacag gc 42

<210> 31
<211> 21
<212> DNA
<213> Artificial Sequence

<220>
<223> Staple oligonucleotide

<400> 31
acaggaaaaa cgctgcagaa g 21

<210> 32
<211> 28
<212> DNA
<213> Artificial Sequence

<220>
<223> Staple oligonucleotide

<400> 32
taaaataaaa gacagcatcg gcaaatca 28

<210> 33
<211> 42
<212> DNA

<213> Artificial Sequence

<220>
<223> Staple oligonucleotide

<400> 33
aagacaatca agagctcaga aagggataag caagagaatt ga 42

<210> 34
<211> 49
<212> DNA
<213> Artificial Sequence

<220>
<223> Staple oligonucleotide

<400> 34
ggcaaaagaa ggcaggatcg tcagggagct gtttaataca tttcgcaaa 49

<210> 35
<211> 42
<212> DNA
<213> Artificial Sequence

<220>
<223> Staple oligonucleotide

<400> 35
atggtaattg tatcatatgc ggccaacatg taataatgcc gg 42

<210> 36
<211> 42
<212> DNA
<213> Artificial Sequence

<220>
<223> Staple oligonucleotide

<400> 36
gtttagataa tcagcgtctg gccttcctaa atttaaaagc ct 42

<210> 37
<211> 42
<212> DNA
<213> Artificial Sequence

<220>
<223> Staple oligonucleotide

<400> 37
taagggatat caccaagtgc cagcctttaa ttgtagaata ga 42

<210> 38
<211> 42
<212> DNA
<213> Artificial Sequence

<220>

<223> Staple oligonucleotide

<400> 38
caaacgtcgc caccaaggat tagtagcgaa ccatcccaat ag 42

<210> 39
<211> 42
<212> DNA
<213> Artificial Sequence

<220>
<223> Staple oligonucleotide

<400> 39
atgttagcga ggaaccgaac atgagtgaga aacagtacat aa 42

<210> 40
<211> 42
<212> DNA
<213> Artificial Sequence

<220>
<223> Staple oligonucleotide

<400> 40
actagaaatg gtttaatttc aaaatgctta ggttgtgaga ag 42

<210> 41
<211> 42
<212> DNA
<213> Artificial Sequence

<220>
<223> Staple oligonucleotide

<400> 41
cccaaatacc agaatccgct ccctaatgag tgagcaatcg gc 42

<210> 42
<211> 42
<212> DNA
<213> Artificial Sequence

<220>
<223> Staple oligonucleotide

<400> 42
gagaaaccaa cgtaatcggc ccgcatcgta accgtttcat ca 42

<210> 43
<211> 49
<212> DNA
<213> Artificial Sequence

<220>
<223> Staple oligonucleotide

<400> 43

caataggaac gccaatgtga gagatgggtc aggaatccca gtctctaga 49

<210> 44
<211> 49
<212> DNA
<213> Artificial Sequence

<220>
<223> Staple oligonucleotide

<400> 44
aactggctca ttatgtaccg tacagccctt tcagcattcg agcttcatt 49

<210> 45
<211> 42
<212> DNA
<213> Artificial Sequence

<220>
<223> Staple oligonucleotide

<400> 45
aacaatgcag atagacgcaa ttccggctga tgcaaatcca at 42

<210> 46
<211> 42
<212> DNA
<213> Artificial Sequence

<220>
<223> Staple oligonucleotide

<400> 46
ctaacggaac gccaagacga ctggatagtt taaacgtgtt tt 42

<210> 47
<211> 42
<212> DNA
<213> Artificial Sequence

<220>
<223> Staple oligonucleotide

<400> 47
aatctacgca tagtaagagc gtttgatggt ggttcgaagg ga 42

<210> 48
<211> 42
<212> DNA
<213> Artificial Sequence

<220>
<223> Staple oligonucleotide

<400> 48
tttagttgaa ataccgaccg tccggaatac cagtaagaat cg 42

<210> 49

<211> 49
<212> DNA
<213> Artificial Sequence

<220>
<223> Staple oligonucleotide

<400> 49
aaccctctcc acagaacact gggaggttca gacggtcgaa atccgcgac 49

<210> 50
<211> 42
<212> DNA
<213> Artificial Sequence

<220>
<223> Staple oligonucleotide

<400> 50
tacagagtgt cgtccatttt cccgccacac gcagtggtgg ca 42

<210> 51
<211> 42
<212> DNA
<213> Artificial Sequence

<220>
<223> Staple oligonucleotide

<400> 51
cagccttaag aaacgcgtct tactcgtaat tagagccgtc aa 42

<210> 52
<211> 42
<212> DNA
<213> Artificial Sequence

<220>
<223> Staple oligonucleotide

<400> 52
aatatataca tataattttg tacattcaga aattaagcca gc 42

<210> 53
<211> 42
<212> DNA
<213> Artificial Sequence

<220>
<223> Staple oligonucleotide

<400> 53
gaatcgtcat aaaagggcga aaggagcggc tttgacgagc aa 42

<210> 54
<211> 42
<212> DNA
<213> Artificial Sequence

<220>
<223> Staple oligonucleotide

<400> 54
agttcagccc gaaaaactaa acaaaagggt cgagaaaacg gg 42

<210> 55
<211> 42
<212> DNA
<213> Artificial Sequence

<220>
<223> Staple oligonucleotide

<400> 55
taatattcat tgaaccaccg agtaaagaga acgtataacg tg 42

<210> 56
<211> 42
<212> DNA
<213> Artificial Sequence

<220>
<223> Staple oligonucleotide

<400> 56
gcatctgcca gtactatatg ttcttctgaa tacataattc at 42

<210> 57
<211> 42
<212> DNA
<213> Artificial Sequence

<220>
<223> Staple oligonucleotide

<400> 57
aaatcaaaca acttcgtaat caggattaaa ttaccagcgc ca 42

<210> 58
<211> 42
<212> DNA
<213> Artificial Sequence

<220>
<223> Staple oligonucleotide

<400> 58
ccggaagtta gagctcttaa aaggcttgca ccctcttcaa cc 42

<210> 59
<211> 42
<212> DNA
<213> Artificial Sequence

<220>
<223> Staple oligonucleotide

<400> 59
gggacgaacg acggccagtt taagacgcgg ttataaggaa ac 42

<210> 60
<211> 42
<212> DNA
<213> Artificial Sequence

<220>
<223> Staple oligonucleotide

<400> 60
aaatatgata aaacagaggt gcattgcaac tatcgaatta ac 42

<210> 61
<211> 42
<212> DNA
<213> Artificial Sequence

<220>
<223> Staple oilgonucleotide

<400> 61
catataatgc tgtacagtat taacaccggg gaacaatatt ac 42

<210> 62
<211> 42
<212> DNA
<213> Artificial Sequence

<220>
<223> Staple oilgonucleotide

<400> 62
ttcatcgcag tgccacgctg atcaatcaag gaatttagat aa 42

<210> 63
<211> 42
<212> DNA
<213> Artificial Sequence

<220>
<223> Staple oilgonucleotide

<400> 63
ttttattcaa gcaagacttg ctgctattcc taattatccc aa 42

<210> 64
<211> 28
<212> DNA
<213> Artificial Sequence

<220>
<223> Staple oilgonucleotide

<400> 64
gcgattaccg gcacaatctt gctttgaa 28

<210> 65
<211> 35
<212> DNA
<213> Artificial Sequence

<220>
<223> Staple oilgonucleotide

<400> 65
tagatttagt ttgaatgttt tggatggcca aactc 35

<210> 66
<211> 42
<212> DNA
<213> Artificial Sequence

<220>
<223> Staple oilgonucleotide

<400> 66
gctcgaggtg aattttaatt gctgaattcc aagcgaacca ga 42

<210> 67
<211> 28
<212> DNA
<213> Artificial Sequence

<220>
<223> Staple oligonucleotide

<400> 67
cgcgttttca tcggcctcag acgccacc 28

<210> 68
<211> 21
<212> DNA
<213> Artificial Sequence

<220>
<223> Staple oligonucleotide

<400> 68
catcctacca agaacgggta t 21

<210> 69
<211> 42
<212> DNA
<213> Artificial Sequence

<220>
<223> Staple oligonucleotide

<400> 69
atagcgatag cttaatatat gaagttacta ataaggcaag cc 42

<210> 70
<211> 42
<212> DNA

<213> Artificial Sequence

<220>
<223> Staple oligonucleotide

<400> 70
aatgcagaac gcgcagcatg tcatttggcc aatgaacaga at 42

<210> 71
<211> 42
<212> DNA
<213> Artificial Sequence

<220>
<223> Staple oligonculeotide

<400> 71
gcctcagagc ataatatgat aagcagcgcg taatgggcgg at 42

<210> 72
<211> 42
<212> DNA
<213> Artificial Sequence

<220>
<223> Staple oligonucleotide

<400> 72
aattaagctg ataattttgc gccaacctgc ggggtgccac cc 42

<210> 73
<211> 42
<212> DNA
<213> Artificial Sequence

<220>
<223> Staple oligonucleotide

<400> 73
gaatccttgc ttctaagtaa gaaatagccc acccttttcc ag 42

<210> 74
<211> 35
<212> DNA
<213> Artificial Sequence

<220>
<223> Staple oligonucleotide

<400> 74
ccatcaaagc taaatcggtt gtaccaaaag acagt 35

<210> 75
<211> 42
<212> DNA
<213> Artificial Sequence

<220>

<223> Staple oligonucleotide

<400> 75
gttctagcaa taaaaaggca aagaattaca ataacttaaa gg 42

<210> 76
<211> 42
<212> DNA
<213> Artificial Sequence

<220>
<223> Staple oligonucleotide

<400> 76
gagccagcca tatttaacaa cttatacaaa tagaaacata aa 42

<210> 77
<211> 28
<212> DNA
<213> Artificial Sequence

<220>
<223> Staple oligonucleotide

<400> 77
taataagttc tgtctcaaca atagataa 28

<210> 78
<211> 42
<212> DNA
<213> Artificial Sequence

<220>
<223> Staple oligonucleotide

<400> 78
gttatctcaa atatcaaacc cgagccagaa gccgttatca tt 42

<210> 79
<211> 28
<212> DNA
<213> Artificial Sequence

<220>
<223> Staple oligonucleotide

<400> 79
tacctttgaa aacagagata actgaaca 28

<210> 80
<211> 42
<212> DNA
<213> Artificial Sequence

<220>
<223> Staple oligonucleotide

<400> 80

accccggcga aagactgctc catgttaaac ctgtagccag ct 42

<210> 81
<211> 42
<212> DNA
<213> Artificial Sequence

<220>
<223> Staple oligonucleotide

<400> 81
gccggcagag ccaagcttgc atgcctgcca cacaaccctg ac 42

<210> 82
<211> 42
<212> DNA
<213> Artificial Sequence

<220>
<223> Staple oligonucleotide

<400> 82
tggtaatatc cacataagag tctgtccatc acgcagcctt gc 42

<210> 83
<211> 42
<212> DNA
<213> Artificial Sequence

<220>
<223> Staple oligonucleotide

<400> 83
gaggaagacc tcccatcaga taacgtcaga attagttcat ta 42

<210> 84
<211> 42
<212> DNA
<213> Artificial Sequence

<220>
<223> Staple oligonucleotide

<400> 84
tataatccgt tagacgcgta ctatggttgg cgctatactg cg 42

<210> 85
<211> 42
<212> DNA
<213> Artificial Sequence

<220>
<223> Staple oligonucleotide

<400> 85
acattaatca aaaataattc gaaaagccca tatgtagagg gt 42

<210> 86

<211> 42
<212> DNA
<213> Artificial Sequence

<220>
<223> Staple oligonucleotide

<400> 86
ttgcgtattg gggaataagc ataaagtgta aagccaggcg gt 42

<210> 87
<211> 42
<212> DNA
<213> Artificial Sequence

<220>
<223> Staple oligonucleotide

<400> 87
caaacaaatt atcaggaatt atcatcatgt ttggatccaa at 42

<210> 88
<211> 42
<212> DNA
<213> Artificial Sequence

<220>
<223> Staple oligonucleotide

<400> 88
agatgatgtt aagccgggag acataaaaat tattttgcca gt 42

<210> 89
<211> 42
<212> DNA
<213> Artificial Sequence

<220>
<223> Staple oligonucleotide

<400> 89
tctgaataag gagcttttgc ggaacaaaag tattatacaa aa 42

<210> 90
<211> 42
<212> DNA
<213> Artificial Sequence

<220>
<223> Staple oligonucleotide

<400> 90
cagctttagt tgggtaacgc cagggtttga tcgcattcat ta 42

<210> 91
<211> 42
<212> DNA
<213> Artificial Sequence

<220>
<223> Staple oligonucleotide

<400> 91
ttatacttca aaattatttg ctcagatgga acacccccac aa 42

<210> 92
<211> 42
<212> DNA
<213> Artificial Sequence

<220>
<223> Staple oligonucleotide

<400> 92
agaaagttag aaccgtacct tcattagacc gttgggaaga aa 42

<210> 93
<211> 49
<212> DNA
<213> Artificial Sequence

<220>
<223> Staple oligonucleotide

<400> 93
tcccttaccg gcgaacgtgg cgagaaagcg aaatcttacg aggttaata 49

<210> 94
<211> 42
<212> DNA
<213> Artificial Sequence

<220>
<223> Staple oligonucleotide

<400> 94
ttgtaaacga cagtacaaag ccgaggcgta gtacctttgc tc 42

<210> 95
<211> 42
<212> DNA
<213> Artificial Sequence

<220>
<223> Staple oligonucleotide

<400> 95
agtcaatagt gaattttaac caataacgcc ttattcctca ga 42

<210> 96
<211> 42
<212> DNA
<213> Artificial Sequence

<220>
<223> Staple oligonucleotide

<400> 96
ccaccagaat ggaacagcca tacagggata gcgtacaata gg 42

<210> 97
<211> 42
<212> DNA
<213> Artificial Sequence

<220>
<223> Staple oligonucleotide

<400> 97
gactttatac atttgaggat taatcaactt tgaagaccgc gc 42

<210> 98
<211> 42
<212> DNA
<213> Artificial Sequence

<220>
<223> Staple oligonucleotide

<400> 98
gaaacaaaat tacctgagca agaaacaaag taacatacca ta 42

<210> 99
<211> 42
<212> DNA
<213> Artificial Sequence

<220>
<223> Staple oligonucleotide

<400> 99
ctttcctagt gaggtccccc tcgttttagg agtgatcggt tt 42

<210> 100
<211> 42
<212> DNA
<213> Artificial Sequence

<220>
<223> Staple oligonucleotide

<400> 100
aaagtttgag taacttcgac atccagagtt gcaccttctg ta 42

<210> 101
<211> 42
<212> DNA
<213> Artificial Sequence

<220>
<223> Staple oligonucleotide

<400> 101
ttcaccacgg ggagtggggt gacaattcag gtcgacacga cg 42

<210> 102
<211> 35
<212> DNA
<213> Artificial Sequence

<220>
<223> Staple oligonucleotide

<400> 102
cttgacgggg aaagtaaatc agatacataa caaca 35

<210> 103
<211> 42
<212> DNA
<213> Artificial Sequence

<220>
<223> Staple oligonucleotide

<400> 103
ttttcttgaa aatcctgtta catcgggaaa cgccagggtg gt 42

<210> 104
<211> 42
<212> DNA
<213> Artificial Sequence

<220>
<223> Staple oligonucleotide

<400> 104
gaattatgat tgcttttaac gacgtaaacc tgattattcc tg 42

<210> 105
<211> 42
<212> DNA
<213> Artificial Sequence

<220>
<223> Staple oligonucleotide

<400> 105
ttcgccttca tttcacatca atttaatgat aaccttgaaa ac 42

<210> 106
<211> 50
<212> DNA
<213> Artificial Sequence

<220>
<223> Staple oligonucleotide with adaptor extension

<400> 106
taacaggatt agcagagcga ggaagaaaaa taatatccaa aatcaccagt 50

<210> 107
<211> 50
<212> DNA

<213> Artificial Sequence

<220>
<223> Staple oligonucleotide with adaptor extension

<400> 107
taacaggatt agcagagcga ggtaccgcac tcatcgaggc ttatccggta 50

<210> 108
<211> 38
<212> DNA
<213> Artificial Sequence

<220>
<223> Staple oligonucleotide with adaptor extension

<400> 108
taacaggatt agcagagcga ggaaaatcgc gcagaggc 38

<210> 109
<211> 50
<212> DNA
<213> Artificial Sequence

<220>
<223> Staple oligonucleotide with adaptor extension

<400> 109
taacaggatt agcagagcga ggacaaaagg taaagtaaag aatataaagt 50

<210> 110
<211> 50
<212> DNA
<213> Artificial Sequence

<220>
<223> Staple oligonucleotide with adaptor extension

<400> 110
taacaggatt agcagagcga ggtctgagag actacctttt atcaaaatca 50

<210> 111
<211> 50
<212> DNA
<213> Artificial Sequence

<220>
<223> Staple oligonucleotide with adaptor extension

<400> 111
taacaggatt agcagagcga ggtgcgtaga ttttcaggtt gaataccaag 50

<210> 112
<211> 50
<212> DNA
<213> Artificial Sequence

<220>

<223> Staple oligonucleotide with adaptor extension

<400> 112
taacaggatt agcagagcga ggaagaacgc gaggcgttaa ttttatcctg 50

<210> 113
<211> 50
<212> DNA
<213> Artificial Sequence

<220>
<223> Staple oligonucleotide with adaptor extension

<400> 113
taacaggatt agcagagcga ggaagccctt tttaagaagt aaatcgtcgc 50

<210> 114
<211> 50
<212> DNA
<213> Artificial Sequence

<220>
<223> Staple oligonucleotide with adaptor extension

<400> 114
taacaggatt agcagagcga gggagcgcta atatcagaaa attaattaca 50

<210> 115
<211> 50
<212> DNA
<213> Artificial Sequence

<220>
<223> Staple oligonucleotide with adaptor extension

<400> 115
taacaggatt agcagagcga ggttaccaac gctaacgaga ttttttgttt 50

<210> 116
<211> 50
<212> DNA
<213> Artificial Sequence

<220>
<223> Staple oligonucleotide with adaptor extension

<400> 116
taacaggatt agcagagcga ggaattcatc aatataatac agaaataaag 50

<210> 117
<211> 50
<212> DNA
<213> Artificial Sequence

<220>
<223> Staple oligonucleotide with adaptor extension

<400> 117

taacaggatt agcagagcga ggtcaaaaat gaaaatagaa gtcagagggt 50

<210> 118
<211> 50
<212> DNA
<213> Artificial Sequence

<220>
<223> Staple oligonucleotide with adaptor extension

<400> 118
taacaggatt agcagagcga ggagaaccaa gccagatttg atgatacagg 50

<210> 119
<211> 50
<212> DNA
<213> Artificial Sequence

<220>
<223> Staple oligonucleotide with adaptor extension

<400> 119
taacaggatt agcagagcga ggaccacgga ataagtttaa agaaacgcaa 50

<210> 120
<211> 50
<212> DNA
<213> Artificial Sequence

<220>
<223> Staple oligonucleotide with adaptor extension

<400> 120
taacaggatt agcagagcga ggaacgttat taattttaat tatcagatga 50

<210> 121
<211> 38
<212> DNA
<213> Artificial Sequence

<220>
<223> Staple oligonucleotide with adaptor extension

<400> 121
taacaggatt agcagagcga ggacaatttc atttgaat 38

<210> 122
<211> 38
<212> DNA
<213> Artificial Sequence

<220>
<223> Staple oligonucleotide with adaptor extension

<400> 122
taacaggatt agcagagcga ggaattaatt ttccctta 38

<210> 123

<211> 50
<212> DNA
<213> Artificial Sequence

<220>
<223> Staple oligonucleotide with adaptor extension

<400> 123
taacaggatt agcagagcga ggcgagaaaa ctttttcacg caagacaaag 50

<210> 124
<211> 50
<212> DNA
<213> Artificial Sequence

<220>
<223> Staple oligonucleotide with adaptor extension

<400> 124
taacaggatt agcagagcga ggcactaaca actaatagtt aaatcctttg 50

<210> 125
<211> 50
<212> DNA
<213> Artificial Sequence

<220>
<223> Staple oligonucleotide with adaptor extension

<400> 125
taacaggatt agcagagcga ggtggcatga ttaagactga atacccaaaa 50

<210> 126
<211> 50
<212> DNA
<213> Artificial Sequence

<220>
<223> Staple oligonucleotide with adaptor extension

<400> 126
taacaggatt agcagagcga ggtaaataag aataaacagt gataaataag 50

<210> 127
<211> 50
<212> DNA
<213> Artificial Sequence

<220>
<223> Staple oligonucleotide with adaptor extension

<400> 127
taacaggatt agcagagcga ggaaggtaaa tattgacgac cgattgaggg 50

<210> 128
<211> 50
<212> DNA
<213> Artificial Sequence

<220>
<223> Staple oligonucleotide with adaptor extension

<400> 128
taacaggatt agcagagcga ggctattatc atggctatgg aaagcgcagt 50

<210> 129
<211> 38
<212> DNA
<213> Artificial Sequence

<220>
<223> Staple oligonucleotide with adaptor extension

<400> 129
taacaggatt agcagagcga ggccattacc attagcaa 38

<210> 130
<211> 50
<212> DNA
<213> Artificial Sequence

<220>
<223> Staple oligonucleotide with adaptor extension

<400> 130
taacaggatt agcagagcga ggcagtaggg cttaattgta aagccaacgc 50

<210> 131
<211> 50
<212> DNA
<213> Artificial Sequence

<220>
<223> Staple oligonucleotide with adaptor extension

<400> 131
taacaggatt agcagagcga ggtcaccttg ctgaacctaa aatatcttta 50

<210> 132
<211> 22
<212> DNA
<213> Artificial Sequence

<220>
<223> Adaptor oligonucleotide

<400> 132
cctcgctctg ctaatcctgt ta 22

<210> 133
<211> 84
<212> DNA
<213> Artificial Sequence

<220>
<223> Hairpin oligonucleotide

<400> 133

tttttttatct acatattttt ttttttatgt agattttttt tttttttttt tttttttttt 60

tttttttttt tttttttttt tttt 84

<210> 134
<211> 75
<212> DNA
<213> Artificial Sequence

<220>
<223> Oligonucleotide Q-T60

<400> 134

ggttggtgtg gttggttttt tttttttttt tttttttttt tttttttttt tttttttttt 60

tttttttttt ttttt 75

<210> 135
<211> 140
<212> DNA
<213> Artificial Sequence

<220>
<223> Oligonucleotide Q-T125

<400> 135

ggttggtgtg gttggttttt tttttttttt tttttttttt tttttttttt tttttttttt 60

tttttttttt tttttttttt tttttttttt tttttttttt tttttttttt tttttttttt 120

tttttttttt tttttttttt 140

<210> 136
<211> 51
<212> DNA
<213> Artificial Sequence

<220>
<223> Staple oligonucleotide with channel modification M1

<400> 136
ccaccagaat ggaacagcca tacagggata gcgtacaata ggaatttccg g 51

<210> 137
<211> 40
<212> DNA
<213> Artificial Sequence

<220>
<223> Staple oligonucleotide with channel modification M1

<400> 137
cccataccag tcaggacaac agaataaggc ttgcatacga 40

<210> 138
<211> 46
<212> DNA
<213> Artificial Sequence

<220>
<223> Staple oligonucleotide with channel modification M2

<400> 138
ccaccagaat ggaacagcca tacagggata gcgtacaatt ttccgg 46

<210> 139
<211> 45
<212> DNA
<213> Artificial Sequence

<220>
<223> Staple oligonucleotide with channel modification M2

<400> 139
aggaacccat accagtcagg acaacagaat aaggcttgca tacga 45

??

??

??

??

1

1

## Description

The present invention relates to nucleic acid based nanopores comprising one or more self assembled nucleic acids. The present invention further relates to nanopore complexes comprising at least one of said nucleic acid based nanopores, to uses of the nucleic acid based nanopores as sensor and for cell-specific targeting, cell-specific labeling and/or cell-specific drug delivery. The present invention further relates to nucleic acid based nanopores and nanopore complexes for use in the diagnosis of diseases and to compositions for obtaining the nucleic acid based nanopores.

BACKGROUND OF THE INVENTION

Proteins and peptides that form channels through lipid bilayer membranes are an abundant class of biological macromolecules found in all three domains of life. The basic function of membrane channels is to facilitate the transport of water, ions, or other entities through otherwise impermeable lipid membranes. Membrane channels provide a means for cells for the exchange of matter and information with the outside world. They are fundamental to processes such as cellular communication, maintenance of membrane potentials, or metabolism.

The natural $\alpha$-hemolysin channel protein has a characteristic mushroom-like shape and is formed by multimerization of subunits (Song et al., 1996).

Nanoscale membrane pores have shown great potential for the use as single molecule biosensors (see e.g. Hall et al., 2010, Branton et al., 2008), whose operation principle is based on the transient blockage of ionic current by analyte molecules. The type of sensing task that can be accomplished with such 'nanopores' depends on their size and their chemical structure. Nanopore sensors based on naturally occurring membrane pores provide excellent electrical properties, but altering the geometry of biological pores and introducing chemical functions through genetic engineering or chemical conjugation is challenging.

*Bell et al.* (2012) disclose hybrid nanopores which are obtained by inserting DNA origami structures into solid-state nanopores. Wei *et al.* (2012) disclose DNA origami gatekeepers for solid-state nanopores. Kaucher *et al.* (2006) disclose an unimolecular G-quadruplex that functions as a synthetic transmembrane $Na^+$ transporter. Akhshi et *al.* (2012) disclose a G-quadruplex DNA channel. Forman *et al.* (2000) disclose a lipophilic G-quadruplex as artificial cation channel.

There is a need in the art for improved nanopores and nanopore complexes, which can be easily adapted and tailored for different uses.

SUMMARY OF THE INVENTION

According to the present invention this object is solved by a nucleic acid based nanopore comprising one or more self-assembled nucleic acids and formed or assembled by the one or more nucleic acids, said self-assembled nucleic acids being obtained via molecular self-assembly of one or more scaffold nucleic acid strand(s) and/or a plurality of staple oligonucleotide strands,
comprising at least one recognition binding moiety,
wherein the nucleic acid based nanopore comprises

(i) a transmembrane domain having a hollow channel and comprising a plurality of helical nucleic acid domains,
(ii) optionally, a cap domain comprising helical nucleic acid domains,

and wherein adhesion or attachment of the nanopore to a lipid bilayer, membrane or lipid vesicle via recognition binding moieties, leads to formation of a transmembrane channel which spans through said lipid bilayer, membrane or lipid vesicle.

According to the present invention this object is solved by a nanopore complex comprising at least one nucleic acid based nanopore according to the invention.

According to the present invention this object is solved by the use of a nucleic acid based nanopore according to the invention as sensor.

According to the present invention this object is solved by the use of a nucleic acid based nanopore or a nanopore complex according to the invention for controlling membrane potentials, transmembrane traffic or interference with membrane functions.

According to the present invention this object is solved by the use of a nucleic acid based nanopore or a nanopore complex according to the invention for cell-specific targeting, cell-specific labeling and/or cell-specific drug delivery.

According to the present invention this object is solved by providing the nucleic acid based nanopore or a nanopore complex according to the invention for the diagnosis of a disease.

According to the present invention this object is solved by a composition for obtaining the nucleic acid based nanopore according to the invention comprising a scaffold nucleic acid strand derived from M 13 phage DNA being a nucleic acid comprising the nucleotide sequence of SEQ ID NO. 1 and/or a plurality of staple oligonucleotide strands which are at least partially complementary to parts of the scaffold nucleic acid strand and are oligonucleotides comprising a nucleotide sequence of SEQ ID NOs. 2 to 131, adaptor oligonucleotides comprising recognition binding moiety(ies), optionally, adaptor oligonucleotides comprising recognition sensing moiety(ies), further component(s) and/or gating mechanism component(s), suitable buffers and salts.

DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention.

Numbers of nucleotides, nucleic acids, components, moieties or other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "15 to 100 nucleotides " should be interpreted to include not only the explicitly recited values of 15 to 100, but also include individual values and sub-ranges within the indicated range. Thus, included in this numerical range are individual values such as 15, 16, 17, 18, 19, ... 97, 98, 99, 100 and sub-ranges such as from 20 to 60, from 15 to 20, from 20 to 40, from 15 to 30 etc. As an illustration, a numerical range of "about 2 to about 100 nm " should be interpreted to include not only the explicitly recited values of 2 to 100, but also include individual values and sub-ranges within the indicated range. Thus, included in this numerical range are individual values such as 2, 3, 4, 5, 6, 7, ... 98, 99, 100 and sub-ranges such as from 2 to 50 nm, 3 to 50 nm, 4 to 50 nm, such as about 42 nm, or sub-ranges such as from 2 to 20 nm, 3 to 20 nm, 3 to 10 nm, 4 to 20 nm or 4 to 10 nm, etc. This same principle applies to ranges reciting only one numerical value. Furthermore, such an interpretation should apply regardless of the breadth of the range or the characteristics being described.

*- Nucleic acid based nanopores*

As described above, the present invention provides nucleic acid based nanopores comprising one or more self-assembled nucleic acids and formed or assembled by the one or more nucleic acids. Said self-assembled nucleic acids are obtained via molecular self-assembly of one or more scaffold nucleic acid strand(s) and/or a plurality of staple oligonucleotide strands.

According to the invention, the nucleic acid based nanopores of the invention comprise at least one recognition binding moiety and comprise

(i) a transmembrane domain having a hollow channel and comprising a plurality of helical nucleic acid domains,
(ii) optionally, a cap domain comprising helical nucleic acid domains.

According to the invention, adhesion or attachment of the nanopore to a lipid bilayer, membrane or lipid vesicle via recognition binding moieties, leads to formation of a transmembrane channel which spans through said lipid bilayer, membrane or lipid vesicle. The term "nanopore" as used herein refers to a structure formed or assembled by nucleic acid molecule(s) which has a hollow channel or hollow tunnel or hollow tube or hole, which provides a connection between two reservoirs or compartments, which are otherwise separate from each other, such as the outside and inside of a vesicle or a cell or an extracellular and an intracellular room. Preferably, the hollow channel/tunnel/tube or hole of a nanopore has a diameter in the nm range, such as about 0.1 nm to about 1,000 nm, e.g. about 0.1 to about 100 nm.

The nanopore of the invention is not a (single) DNA double helix or nucleic acid double helix.

Preferably, the nanopore of the invention is or becomes or is intended for becoming associated with a (cell) membrane, lipid bilayer or lipid vesicle.

A nanopore as known in the art may, for example, be created by a pore-forming protein, such as alpha hemolysin and MspA porin, or is a hole in synthetic materials, such as silicon or graphene.

The nanopores of the present invention are nucleic acid based nanopores, i.e. they are formed by nucleic acids and not by pore-forming proteins. The nucleic acid based nanopores according to the invention have a hollow channel/tunnel/tube which is formed by the nucleic acids and not by proteins or protein domains.

The nucleic acids of the nucleic acid based nanopores according to the invention preferably comprise

DNA,
RNA,
modified and artificial nucleic acids and nucleic acid analogues, such as

- nucleic acids comprising modified backbones such as peptide nucleic acids (PNA), Morpholino and locked nucleic acids (LNA), glycol nucleic acids (GNA), threose nucleic acids (TNA),
- nucleic acids comprising modified nucleotides or bases or base analogues or artificial nucleobases,
- nucleic acids comprising abasic and other synthetic residues,

or combinations thereof.

In embodiments of the invention, a nanopore can comprise nucleic acid molecules which are DNA which is partially modified, such as comprising parts without bases (abasic) and parts with modified backbones and/or bases.
The intended use or application of the nanopore of the invention can have influence on the choice of nucleic acid(s) or modified nucleic acid(s) to be used for obtaining the respective nanopore.
According to the invention, the nucleic acid based nanopores of the invention comprise self assembled nucleic acid(s), such as self assembled DNA molecule(s).
Preferably, the one or more nucleic acids of the nanopore of the invention comprise a plurality of helical nucleic acid domains which form the structure of and/or assemble the nanopore.
The term "a plurality of helical nucleic acid domains" as used herein refers to several helical nucleic acid domains, which are at least two helical nucleic acid domains, preferably 2 to 100, such as 10 to 70, and can be as high as several 100 or even several 1,000 helical nucleic acid domains.
In an example, a nanonopore comprises 54 double helices or helical nucleic acid domains.
In one embodiment, the nucleic acid based nanopores of the invention comprises or consists of a plurality of nucleic acids.
The term "a plurality of nucleic acids" as used herein refers to several nucleic acids, which are at least two nucleic acids, preferably at least three, and can be as high as several 100 or several 1,000 nucleic acid(s) / nucleic acid molecule(s).
In one embodiment, the plurality of nucleic acids that form the nanopore of the invention can be chemically ligated after formation/generation of the nanopore, such that the nanopore than comprises only one or several (such as 2, 3, 4, 5 up to e.g. 10) nucleic acid molecules.
The nucleic acid based nanopores according to the invention comprise

(i) a transmembrane domain,
(ii) optionally, a cap domain.

The *transmembrane domain* of the nucleic acid based nanopores according to the invention have a hollow channel or hollow tunnel or hollow tube or hole, such as a centrally located hollow channel or hollow tunnel or hollow tube or hole.
The transmembrane domain of the nucleic acid based nanopores according to the invention comprises helical nucleic acid domains.
The helical nucleic acid domains of the transmembrane domain are preferably parallel helical nucleic acid domains.
The helical nucleic acid domains of the transmembrane domain are preferably DNA double helices, such as 6 DNA double helices.
The hollow channel formed by the transmembrane domain or of the transmembrane domain has preferably a length of about 2 to about 200 nm.
The preferred length of the hollow channel formed by the transmembrane domain or of the transmembrane domain depends on the intended use of the nanopore.
In one embodiment, the hollow channel has at least or about the length or thickness of a lipid bilayer, which is about 4 nm thick.
The hollow channel can also have a length of about 2 nm, in particular in an embodiment where a nanopore of the invention can form dimers or multimers which then span a lipid bilayer of (cell) membrane.
In one embodiment, the hollow channel is longer, such nanopores are preferably suitable for sequencing or filtering applications.
In one embodiment, the hollow channel has a length of about 2 to about 100 nm, preferably 2 to 50 nm or about 3 to 50 nm or about 4 to 50 nm, such as about 42 nm. In one embodiment, the hollow channel has a length of about 2 to about 20 nm, such as about 3 to 20 nm, about 3 to 10 nm, about 4 to 20 nm or about 4 to 10 nm.

The hollow channel formed by the transmembrane domain or of the transmembrane domain has preferably a diameter of about 0.1 to 100 nm, preferably about 0.2 to 10 nm, such as about 2 nm.

The preferred diameter of the hollow channel depends on the intended application of the nanopore, such as on the size of the analyte or single molecule to be detected or filtered.

In one embodiment, the hollow channel is modified.

For example, single stranded nucleic acid(s) or oligonucleotide(s) are protruding into the hollow channel or are lining the hollow channel walls.

Said single stranded nucleic acid(s) or oligonucleotide(s) can comprise DNA, RNA, modified nucleic acids, artificial nucleic acids, nucleic acid analogues or combinations thereof, as described above.

Such modification of the hollow channel is preferably for varying the conductivity of the nanopore.

The *cap domain* of the nucleic acid based nanopores according to the invention, if present, comprise helical nucleic acid domains.

The cap domain can be barrel-shaped.

The helical nucleic acid domains of the cap domain are preferably parallel helical nucleic acid domains.

The helical nucleic acid domains of the cap domain are preferably DNA double helices, such as 48 DNA double helices.

Preferably, the adhesion or attachment of the nanopore according to the invention to a lipid bilayer, membrane or lipid vesicle (preferably via recognition binding moieties), leads to formation of a transmembrane channel which spans through said lipid bilayer, membrane or lipid vesicle.

The cap domain preferably adheres to the outside of a cell membrane, i.e. the cis-side of the membrane or lipid bilayer/vesicle.

According to the invention, the nucleic acid based nanopores of the invention furthermore comprises at least one *recognition binding moiety.*

The at least one recognition binding moiety is preferably

- a hydrophobic moiety, preferably for binding to lipid or membrane surfaces, such as cholesterol or its derivatives, lipid(s), fatty acid(s), triglyceride(s),
- a moiety specifically recognizing receptors or other surface molecules of membranes or (target) cell membranes,

  such as
  antibody or fragments thereof,
  receptor ligand or fragments thereof, receptor fragments,
  nucleic acid(s), such as aptamers,
  antigen or epitopes.

The recognition binding moiety/moieties are preferably attached to at least one of the nucleic acids of the nanopore of the invention, such as to the transmembrane domain and/or the cap domain.

The nanopores of the invention can comprise one recognition binding moiety or more than one recognition binding moiety, such as two , three, four and so one, such as 100.

In one example, a nanopore of the invention comprises 26 cholesterols.

The nanopores of the invention can also comprise different recognition binding moieties, such as different hydrophobic moieties and/or different moieties specifically recognizing receptors or other surface membrane molecules.

In a preferred embodiment, the nucleic acid based nanopores of the invention furthermore comprises at least one *recognition sensing moiety.*

Said recognition sensing moiety is a moiety specifically recognizing or binding or interacting with an analyte or molecule which passes through the nanopore or hollow channel.

Such as

antibody or fragments thereof,
receptor ligand or fragments thereof, receptor fragments,
nucleic acid(s), such as aptamers,
antigen or epitopes,

This embodiment is preferably useful when the nanopores of the invention are used as single molecule sensor or for transmembrane traffic.

The recognition sensing moiety/moieties are preferably attached to at least one of the nucleic acids of the nanopore of the invention, preferably to the transmembrane domain, more preferably within the hollow channel of the transmembrane domain.

The nanopores of the invention can comprise one recognition sensing moiety or more than one recognition sensing moiety, such as two, three, four and so one, such as 100.

The nanopores of the invention can also comprise different recognition sensing moieties, such as different antigens and/or aptamers, such as for recognizing different analytes/molecules to pass through the nanopore.

In a preferred embodiment, the nucleic acid based nanopores of the invention furthermore comprise one or more *further components.*

The one or more further components are preferably

- label or tag,
  such as fluorescent dye, chromophore, isotope, quantum dot, ...
- protein or peptide,
  such as an enzyme or its binding and/or catalytic domain, antibody or fragments thereof, receptor ligand or fragments thereof, receptor fragments, antigen or epitopes, preferably to be delivered to a target cell,
- drug,
  such as a drug to be delivered to a target cell,
  such as antimicrobial or anti-viral drugs, anti-cancer drugs,
- nanoparticle or nanocrystal,
- glycosylated or glycosylation label or tag,
  such as glycan, glycoprotein, glycolipid, proteoglycan,
- component for a functional mechanism,

  such as
  a rotor domain enabling the nanopore to move or rotate in the lipid bilayer or membrane,
  an ion pump,
  transport proteins or domains thereof.

The one or more further components are preferably attached to at least one of the nucleic acids of the nanopore of the invention, such as to the transmembrane domain and/or the cap domain.

The nanopores of the invention can comprise one or more further components, such as two, three, four and so one, such as 100.

The nanopores of the invention can also comprise different further components, such as labels/tags and drugs; or labels/tags and rotor domain(s).

Rotor domains enabling the nanopore to move or rotate in a lipid bilayer or membrane can be, for example, similar to a rotor domain of the ATP synthase complex (Boyer 1997).

In a preferred embodiment, the nucleic acid based nanopores of the invention furthermore comprise *gating mechanism component(s)* for opening and closing the nanopore.

Such gating mechanism component(s) can be reactive to binding of ligand(s), such as antigen(s).

For example, gating mechanism component(s) based on aptamers that are capable of binding to antigen(s). The nanopore can be closed, when said aptamer is hybridized to a respective complementary nucleic acid strand, and the nanopore can be open, upon antigen binding. In another exemplary embodiment, the nanopore opens due to a comformational change an aptamer undergoes upon antigen binding.

Further gating mechanism component(s) can be reactive to pressure, temperature, voltage and/or light.

For example:

- mechano-sensitive nucleic acids, such as mechano-sensitive DNA,
- components with a temperature-dependent secondary structure
- components conjugated with temperature-dependent polymers, such as p-NIPAM,
- movable charged structures, which move in an electric field and thereby open/close the nanopore, i.e. the hollow channel,
- photo-switchable molecules, which are e.g. conjugated to nucleic acids

The gating mechanism component(s) are preferably attached to at least one of the nucleic acids of the nanopore of the invention, such as to the transmembrane domain and/or the cap domain.

Preferably, the recognition binding moiety(ies), the recognition sensing moiety(ies), the further component(s) and/or the gating mechanism component(s) comprise adaptor oligonucleotide(s) with a nucleotide sequence which hybridizes to at least one of the nucleic acids of the nanopore of the invention or parts thereof.

Preferably, an adaptor oligonucleotide is covalently attached to said recognition binding moiety(ies), recognition sensing moiety(ies), further component(s) and/or gating mechanism component(s).

According to the invention, the nucleic acid based nanopores of the invention comprise or consist of self-assembled nucleic acids.

According to the invention, the self-assembled nucleic acids are obtained via molecular self-assembly.

The term "molecular self-assembly", as used herein, refers to the process by which nucleic acid molecules adopt a defined arrangement without guidance or management from an outside source. There are two types of self-assembly, intramolecular self-assembly and intermolecular self-assembly. The molecular self-assembly can also be called folding. The assembly of the nucleic acid molecules is directed through noncovalent interactions (e.g., hydrogen bonding, hydrophobic forces, van der Waals forces, $\pi$-$\pi$ interactions, and/or electrostatic) as well as electromagnetic interactions. Molecular self-assembly is an important aspect of bottom-up approaches to nanotechnology. Using molecular self-assembly the final (desired) structure is programmed in the shape and functional groups of the molecules. The nucleic acids, such as DNA, are used as a structural material rather than as a carrier of biological information, to make the structures.

The molecular self-assembly of the nucleic acids according to the invention can be via DNA origami molecular self-assembly of scaffold nucleic acid strand(s) and a plurality of staple or helper oligonucleotide strands.

The molecular self-assembly of the nucleic acids can be via molecular self-assembly of one or more scaffold nucleic acid strand(s).

The molecular self-assembly of the nucleic acids can also be via molecular self-assembly of a plurality of oligonucleotide strands.

The term "DNA origami molecular self-assembly", as used herein, refers to intramolecular folding of a single-stranded DNA scaffold molecule with DNA staple or helper oligonucleotides into a specific molecular structure.

DNA origami structures incorporate DNA as a building material to make nanoscale shapes. In general, the DNA origami process involves the folding of one or more long, "scaffold" DNA strands into a particular shape using a plurality of rationally designed "staple" or "helper" DNA or oligonucleotide strands. The sequences of the staple or "helper"strands are designed such that they hybridize to particular portions of the scaffold strands and, in doing so, force the scaffold strands into a particular shape.
Methods useful in the making of DNA origami structures can be found, for example, in Rothemund 2006, Douglas et al 2009-2; Dietz et al, 2009 or US 7,842,793 B2 (Rothemund). Staple design can be facilitated using, for example, CADnano software, available at http://www.cadnano.org.

Preferably, the scaffold nucleic acid strand(s) are single stranded DNA polynucleotide strand(s).

The scaffold strand or scaffold nucleic acid can have any sufficiently non-repetitive sequence.

For example, in certain embodiments, the scaffold strand is derived from a natural source, such as derived from M13 phage DNA, such as the M13mp18-derived sequence provided in nucleotide sequence of SEQ ID NO. 1. In one embodiment, the scaffold strand comprises or consists of a nucleic acid with SEQ ID NO. 1.

The scaffold strand or scaffold nucleic acid can be artificial.

The scaffold strand or scaffold nucleic acid(s) has/have a length of preferably several thousand nucleotides, such as about 100 to about 100.00 nucleotides, e.g. about 4,000 to about 8,000 nucleotides.

Preferably, the staple or helper oligonucleotide strands are at least partially complementary to parts of the scaffold nucleic acid strand(s).

Preferably, the number of staple or helper oligonucleotide strands are in the range of about 1 to 1,000, e.g. about 50 to about 500.

Preferably, the staple or helper oligonucleotide strands are oligonucleotides comprising or consisting of a nucleotide sequence of SEQ ID NOs. 2 to 131.

In an embodiment, oligonucleotides comprising or consisting of a nucleotide sequence of SEQ ID NOs. 2 to 105 are staple or helper oligonucleotides; and oligonucleotides comprising or consisting of a nucleotide sequence of SEQ ID NOs. 106 to 131 are staple or helper oligonucleoties to which adaptor oligonuleotide(s) can hybridize / are complementary to adaptor oligonucleotide(s), e.g. in that said oligonucleotides comprise single-stranded adaptor extensions to which the adaptor oligonucleotide(s) can hybridize/are complementary to.

The staple or helper oligonucleotides have preferably a length of about 10 to 1,000 nucleotides, such as about 15 to 100 nucleotides, such as about 20 to 60.

In one embodiment, the adaptor oligonucleotides comprised in the recognition binding moiety(ies), the recognition sensing moiety(ies), the further component(s) and/or the gating mechanism component(s) are selected from oligonucleotides comprising a nucleotide sequence of SEQ ID NO. 132.

The number of adaptor oligonucleotides depends on the number and/or kind of recognition binding moiety(ies), the recognition sensing moiety(ies), the further component(s) and/or the gating mechanism component(s) that are comprised in a respective embodiment of the nanopore of the invention.

The adaptor oligonucleotides have preferably a length of about 10 to 1,000 nucleotides, such as about 15 to 100 nucleotides, such as about 15 to 60 or 20 to 60.

In one embodiment, a nucleic acid based nanopore according to the invention comprises the following nucleic acids:

- one scaffold strand, such as the M13mp18-derived sequence provided in nucleotide sequence of SEQ ID NO. 1;
- at least or up to 130 staple or helper oligonucleotide strands, such as oligonucleotides comprising or consisting of a nucleotide sequence of SEQ ID NOs. 2 to 131;
- at least or up to 26 adaptor oligonucleotide comprising recognition binding moieties, such as oligonucleotides each comprising or consisting of a nucleotide sequence of SEQ ID NOs. 132.

In one embodiment, a M13 phage-derived 7249 bases-long single-stranded DNA was folded by 156 DNA oligonucleotides (130 staple oligonucleotides plus 26 adaptor oligonucleotides with cholesterol) into a structure consisting of 54 parallel DNA double-helices close-packed on a honeycomb lattice. See, for example Figure 1a.

Castro et al., 2011, for example, describe close-packing of DNA double helical domains onto a honeycomb lattice or onto a square lattice.

*- Nanopore complexes*

As described above, the present invention provides nanopore complexes.

The nanopore complexes of the invention comprise at least one nucleic acid based nanopore according to the invention.

The nanopore complexes of the invention comprise preferably two or more, such as three, four, five and more of said nucleic acid based nanopores.

The nucleic acid based nanopores of the nanopore complex can differ in their recognition binding moieties, further component(s) and/or gating mechanisms.

The nucleic acid based nanopores of the nanopore complex can differ in their nucleic acid sequences, number of nucleic acid molecules, number of helical domains.

The nucleic acid based nanopores of the nanopore complex can differ in their length and diameter of hollow channel/tunnel/hole.

*Uses of the nucleic acid based nanopores and nanopore complexes*

As described above, the present invention provides uses of a nucleic acid based nanopore of the invention.

Preferably, the nucleic acid based nanopore of the invention is used as sensor, preferably single molecule sensor.

The uses of the nucleic acid based nanopore of the invention comprises

- nucleic acid sequencing,
- sensing nucleic acid secondary structure,
- detection of analytes, such as small molecules, toxins, proteins,
- detection of polymers (such as nanopore mass spectrometry),
- separation of components across a lipid bilayer or a (cell) membrane,
- separation of charges across a lipid bilayer or a (cell) membrane,
- molecular filters and sieves.

The nanopores of the invention can be employed as sensing devices to discriminate analyte molecules, for example, by studying their translocation characteristics.

For example, a nanopore of the invention can be used for single molecule studies of DNA hairpin unzipping and guanine quadruplex unfolding. Single-stranded DNA fits through the central pore of a nanopore having a 2 nm wide central pore, while larger DNA secondary structures such as hairpins or quadruplexes are not. In order to translocate through the DNA channel, the structures need to unzip or unfold, thus providing a characteristic time delay in the current blockades. See, for example, Figure 3.

The nanopores of the invention can be used for separating charges across a lipid bilayer or a (cell) membrane, such as in form of nanobatteries.

As described above, the present invention provides the use a nucleic acid based nanopore of the invention or of a nanopore complex of the invention for

- controlling membrane potentials,
- transmembrane traffic
  and/or

- interference with membrane functions.

Said uses comprise *in vitro* and *in vivo* uses.

Transmembrane traffic comprises, for example, the translocation or transport of nucleic acid molecules or proteins or other molecules through a nanopore of the invention.

See for example, Figure 3 and Example 2.7.

Interference with membrane functions comprises, for example, the formation of holes, i.e. transmembrane channels, via adhesion or attachment of one or more nanopores or nanopore complexes of the invention to a respective membrane.

The uses of the invention comprises voltage gating, pressure, gating, ligand-gating, or gating by other physical effects, such as light, temperature.

As described above, the present invention provides the use of a nucleic acid based nanopore of the invention or of a nanopore complex of the invention for cell-specific targeting, cell-specific labeling and/or cell-specific drug delivery.

In one embodiment, a nanopore of the invention comprises a respective recognition binding moiety specific for target cells and is, thus, suitable for said cell-specific targeting.

In one embodiment, a nanopore of the invention comprises a respective recognition moiety specific for target cells as well as label(s)/tag(s) and is, thus, suitable for said cell-specific labeling.

In one embodiment, a nanopore of the invention comprises a respective recognition binding moiety specific for target cells as well as drug(s) and is, thus, suitable for said cell-specific drug delivery.

As described above, the present invention provides a nucleic acid based nanopore of the invention or of a nanopore complex of the invention for the diagnosis of a disease.

The diseases are, for example,

cancer,
infectious diseases, such as bacterial and/or viral infections,
allergies,
metabolic diseases, such as diabetes,
obesity and heart diseases.

For example, a nanopore of the invention can comprise a respective antibiotic drug or antiviral drug and is, thus, suitable for the prevention and/or treatment of a bacterial infection or viral infection.

For example, a nanopore of the invention can comprise a suitable further component, such as a respective drug and/or an ion pump, and is, thus, suitable for the prevention and/or treatment of heart diseases and/or metabolic diseases, such as diabetes.

For example, a nanopore of the invention can comprise a suitable label or tag and is, thus, suitable for the diagnoses of a disease.

For example, a nanopore of the invention (furthermore) comprises a respective recognition binding moiety specific for cancer cells and is, thus, suitable for the diagnosis of the specific cancer.

The diagnosis of a disease can comprise the cell-specific targeting of diseased cells, tissues and organs; cell-specific labelling of diseased cells, tissues and organs; and/or cell-specific drug delivery to diseased cells, tissues and organs, wherein the diseased cells, tissues and organs are preferably tumor related. The nanopores of the invention will then comprise suitable recognition binding moieties and further components, such as label, drugs.

The cell-specific targeting comprises the recognition and attachment of the nanopore to specific target cells. The cell-specific targeting can further comprise the penetration of the cell membrane and formation of multiple holes/pores into the cell membrane of the target cells, which can result in the killing of said target cells and/or in making said target cells recognizable/susceptible for the immune system of the patient.

For example, the nanopores of the invention can be used for targeting of cancer or tumor cells. The nanopore can be designed to be specifically recognizing and targeting tumor cells by comprising suitable recognition binding moieties which are tumor specific. The nanopore can be utilized for the treatment of a specific tumor in that the nanopore recognizes the specific tumor cells, attached to and then penetrates the tumor cell membrane which results in the killing of said tumor cells and/or in making said tumor cells recognizable/susceptible for the immune system of the patient. The nanopore can furthermore comprise drug(s) and/or further components, as described herein, to be delivered to or into the tumor cells.

*Compositions for obtaining the nanopores*

As described above, the present invention provides compositions for obtaining a nucleic acid based nanopore of the present invention.

A composition of the invention comprises

a scaffold nucleic acid strand derived from M13 phage DNA being a nucleic acid comprising the nucleotide sequence of SEQ ID NO. 1 and/or a plurality of staple oligonucleotide strands which are at least partially complementary to parts of the scaffold nucleic acid strand and are oligonucleotides comprising a nucleotide sequence of SEQ ID NOs. 2 to 131 as defined herein,
adaptor oligonucleotides comprising recognition binding moiety(ies) as defined herein, optionally, adaptor oligonucleotides comprising recognition sensing moiety(ies) as defined herein,
optionally, oligonucleotides comprising further component(s) and/or gating mechanism component(s) as defined herein,
wherein the adaptor oligonucleotides comprised in the recognition binding moiety(ies), the recognition sensing moiety(ies), the further component(s) and/or the gating mechanism component(s) are selected from oligonucleotides comprising a nucleotide sequence of SEQ ID NO. 132,
suitable buffers and salts.

A multitude of experimental obstacles and difficulties were to be overcome in the design and development of the nucleic based nanopore according to the invention, which can also be seen in greater detail from the description of a preferred embodiments as well as the examples and figures.

- The architecture and structure of the nanopore itself had to be designed wherein a multitude of requirements had to be realized, such as stability of the structure, ability to attach to a lipid layer/membrane and then to penetrate said lipid layer/membrane.

- The correct molecular self-assembly of the rather large structure of a nucleic acid based nanopore had to be ensured: by screening and tuning multiple reaction conditions, such as temperature and salt concentrations, the self-assembly of more than 131 nucleic acid molecules into a multitude of neighboring DNA helices had to be adjusted.

- The recognition binding moiety had to be screened and tested, including the attachment points on the nanopore, which are e.g. important for the ability of the nanopore to successfully attach to a lipid layer/membrane.

- The insertion of the nanopore into a lipid bilayer/membrane had to be ensured by choosing the architecture/geometry of the nanopore, the recognition binding moiety, its attachment points, the dimensions of the nanopore.

*Preferred embodiment*

Proteins and peptides that form channels through lipid bilayer membranes are an abundant class of biological macromolecules found in all three domains of life. The basic function of membrane channels is to facilitate the transport of water, ions, or other entities through otherwise impermeable lipid membranes. Membrane channels provide a means for cells for the exchange of matter and information with the outside world. They are fundamental to processes such as cellular communication, maintenance of membrane potentials, or metabolism. Here, the inventors report on a new type of synthetic membrane channel with user-defined geometric specifications that is constructed entirely from DNA. The inventors show that these synthetic channels can be incorporated into lipid bilayer membranes and the inventors study their electrical properties by single-channel electrophysiological measurements. The inventors find remarkable similarities to the behaviour of biological ion channels, such as channel gating caused by molecular fluctuations within the channel. In single molecule translocation experiments with DNA, the inventors highlight one of many potential applications of the synthetic channels, namely as single-molecule sensing devices. Other applications such as the control of membrane potentials, transmembrane traffic, or interference with membrane functions, both in vitro as well as in vivo, are readily conceivable.

The shape of the inventors' synthetic membrane channel is inspired by the natural α-hemolysin channel protein that has a characteristic mushroom-like shape (Song et al., 1996). In contrast to α-hemolysin, however, our channel does not form by multimerization of subunits. We constructed the channel using molecular self-assembly with scaffolded DNA origami (Rothemund 2006, Douglas, et al, 2009-2, Andersen et al., 2009, Dietz et al., 2009, Seeman et al., 2009, Han et al., 2011, Castro et al., 2011).
M13 phage-derived 7249 bases-long single-stranded DNA was folded by 156 DNA oligonucleotides into a structure consisting of 54 parallel DNA double-helices close-packed on a honeycomb lattice (Douglas, et al, 2009-2, Castro et al., 2011) (see e.g. Fig. 1a). The channel consists of two modules/domains: a stem that penetrates and spans a lipid membrane (i.e. the transmembrane domain), and a barrel-shaped cap that adheres to the (cis)-side of the membrane (i.e. the cap domain). Adhesion to the lipid bilayer is mediated by 26 cholesterol moieties that are attached to the cis-facing surface of the barrel (see e.g. Fig. 1a). The stem protrudes centrally from the barrel and consists of six double-helical DNA domains that form a hollow tube. The interior of this tube acts as a transmembrane channel with a diameter of ≈ 2 nm and a length of ≈ 42 nm and runs through both stem and barrel (see e.g. Fig. 1b, c). Transmission electron microscopy (TEM) images taken from purified structures (see e.g. Fig. 1d) confirm that the intended shape is realized. Experiments with unilamellar lipid vesicles show that the synthetic DNA channels bind to lipid bilayer membranes (see e.g. Fig. 1e-g) in the desired orientation in which the cholesterol-modified face of the barrel forms tight contact with the membrane and the stem protrudes into the lipid bilayer (see e.g. Fig. 1f). These observations show that the synthetic DNA channels are capable of forming membrane pores as designed.

In order to demonstrate the electrical conductivity of the resulting membrane pores, the inventors performed single-channel electrophysiological experiments (Sakman & Neher, 1995) using an integrated chip-based setup (Baaken et al., 2008). The inventors added synthetic DNA channels at low concentrations (≈ 200 pM) to the cis side of the setup and applied voltage pulses to facilitate incorporation into the membrane (see e.g. Fig. 9a). As with biological channels, successful membrane incorporation of individual synthetic DNA channels manifested itself in a stepwise increase in transmembrane current (see e.g. Fig. 2a) along with an increase in electrical noise (see e.g. Fig. 9b). Depending on the preparation method, the inventors also observed incorporation of multiple DNA channels into the same membrane (see e.g. Fig. 10). The synthetic DNA channels displayed an average Ohmic conductance of $G = 0.87 \pm 0.15$ nS (see e.g. Fig. 2b,c) per channel in a solution containing 1 M KCl and 2mM $MgCl_2$. A simple geometrical model (Hille 2001) predicts G = 0.78nS for a channel with diameter 2 nm and length 42 nm (see Examples for further details) which agrees favourably with what the inventors observe.

Many natural ion channels display current gating caused by switching between distinct channel conformations with different conductances (Hille 2001). The inventors found that the synthetic DNA channels also displayed gating behavior (see e.g. Fig. 2d, trace i-iii). The inventors hypothesized that stochastic unzipping and rezipping of double-helical DNA domains in the channel may contribute to the observed current gating. To test this idea, the inventors designed two channel 'mutants' that differed from the 'wildtype' channel only by a single-stranded heptanucleotide protruding from the central transmembrane tube (see e.g. Fig. 1b, see Examples for further details). The inventors found that the mutant channels showed more pronounced gating than wildtype channels (see e.g. Fig. 2d, trace iv-vi), and they also significantly differed in their gating time statistics (see e.g. Fig. 2e and 12). Every investigated mutant channel displayed gating, while some of the wildtype channels did not show gating at all (see e.g. Fig. 2d, trace i). These results show that the transmembrane current depends on fine structural details of the synthetic DNA channel.

In the past, nanoscale membrane pores have shown great potential for the use as single molecule biosensors (see e.g. Hall et al., 2010, Branton et al., 2008), whose operation principle is based on the transient blockage of ionic current by analyte molecules. The type of sensing task that can be accomplished with such 'nanopores' depends on their size and their chemical structure. Nanopore sensors based on naturally occurring membrane pores provide excellent electrical properties, but altering the geometry of biological pores and introducing chemical functions through genetic engineering or chemical conjugation is challenging. By contrast, the geometry of synthetic DNA objects (Bell et al., 2012, Wei et al., 2012) and their chemical properties can be tailored for custom nanopore sensing applications.

Here, the inventors utilize their synthetic DNA lipid membrane channel for single molecule studies of DNA hairpin unzipping and guanine quadruplex (Burge et al., 2006) unfolding. Single-stranded DNA is expected to fit through the 2 nm wide central pore of the DNA channel, while larger DNA secondary structures such as hairpins or quadruplexes are not. In order to translocate through the DNA channel, the structures need to unzip or unfold as in similar experiments with α-hemolysin, thus providing a characteristic time delay in the current blockades that reveals the kinetics of structural transitions (Mathe et al., 2004).

For one set of experiments, the inventors used a DNA hairpin with a 9 base pair long stem flanked by 50 thymidines on the 3' end and 6 thymidines on the 5' end (see e.g. Fig. 3a). The hairpin molecules were initially added to the cis side of a lipid membrane containing a single synthetic DNA channel that displayed a stable current baseline without gating. Application of a positive voltage bias leads to capture, unzipping, and translocation of the hairpin structures resulting in transient current blockades (see e.g. Fig. 3a,b). Reversal of the bias after approximately ~30 minutes again led to transient current blockades, this time caused by molecules that had accumulated in the trans compartment by previous translocation through the DNA channel. The blockade amplitudes for both translocation directions were $\Delta I_{cis\text{-}trans} = 11.9 \pm 2.7$ pA and $\Delta I_{trans\text{-}cis} = 20.3 \pm 4.2$ pA. The blockade dwell times were distributed exponentially with a characteristic lifetime of $\tau_{cis\text{-}trans}$ = 1.5 ms and $\tau_{trans\text{-}cis}$ = 1ms, respectively (see e.g. Fig. 3c).

In another set of experiments, the inventors added quadruplex-forming oligonucleotides with a 60 thymidine long single-stranded tail (Q-T60) to the cis side of a membrane containing a single synthetic DNA channel (see e.g. Fig. 3d,e). Again, the inventors observed transient current blockades, which correspond to the capture and threading of quadruplex DNA molecules into the channel, followed by unfolding and subsequent translocation through the pore. Removal of the analyte from the cis compartment restored a stable baseline current without blockades. Subsequent addition of quadruplex DNA with a longer $(dT)_{125}$tail (Q-T125) led to larger current blockades. The average current blockades were $\Delta I_{Q\text{-}T60} = 5.6 \pm 1.0$ pA and $\Delta I_{Q\text{-}TI25} = 15.3 \pm 2.3$ pA, respectively. The larger current blockade for Q-T125 as compared to Q-T60 translocations can be explained by the larger volume occupied in the channel by the longer $T_{125}$ tail as compared to the $T_{60}$ tail (see the Examples for further details). The blockade dwell times are distributed exponentially with characteristic lifetimes of $\tau_{Q\text{-}T60}$ = 9.7 ms and $\tau_{Q\text{-}T125}$ = 8.1 ms.

Therefore, similar to biological pores our synthetic DNA channels can be employed as sensing devices to discriminate analyte molecules by studying their translocation characteristics. Moreover, it is easily conceivable that DNA-based channels can be tailored to provide highest sensitivity and optimum discrimination for a wide range of different molecular targets.

To conclude, the inventors have reported a novel DNA-based synthetic membrane channel. In contrast to biological ion channels, geometry and chemical details of the synthetic channel are user-defined. The inventors have incorporated the channel into lipid bilayers and verified ion flux through the channel. The inventors found quantized conductance values and current gating that depended on channel layout details. The inventors have demonstrated one potential use of the synthetic DNA channel as a single-molecule sensing device. Future modifications of our current ion channel design may find use for more complex sensing tasks and characterization of other types of analyte. The synthetic DNA channels introduced here constitute an entirely new class of biologically-active ion channels. They open up perspectives for applications as targeted antimicrobial agents or delivery means when supplemented with cell-specific recognition mechanisms.

The following examples and drawings illustrate the present invention without, however, limiting the same thereto.

BRIEF DESCRIPTION OF THE DRAWINGS

**Figure 1.** *Synthetic DNA Membrane channels.*

**a** Schematic illustration of the channel formed by 54 double-helical DNA domains packed on a honeycomb lattice. Cylinders indicate double-helical DNA domains, rings indicate mechanical domain limits determined using CanDo (Maarel 2007). Dark grey: transmembrane stem. Light grey strands with light grey ellipsoids indicate cholesterol-modified oligonucleotides that hybridize to single-stranded DNA adaptor strands as indicated.
**b** Geometric specifications of the transmembrane channel. Length L = 47 nm, tube diameter D = 6 nm, inner diameter d = 2 nm. The length of the central channel fully surrounded by DNA helices is 42 nm. The star symbol indicates the position of a 7 base strand extension acting as a 'defect' in channel 'mutants' (1=15 nm for mutant M1 and 1=14 nm for mutant M2).
**c** Cross-sectional view through the channel when incorporated in a lipid bilayer.
**d** Averaged negative-stain TEM images obtained from purified DNA channel structures.
**e,f** TEMimages ofDNA channels adhering to small unilamellar vesicles (SUVs).
**g** TEM image of DNA channels binding to an extended lipid bilayer in the upper right part of the image. DNA channels are found predominantly on lipid covered areas or sticking to SUVs.

**Figure 2.** *Electrical characterization.*

**a** Stepwise increase in ionic current during an incorporation event at V=200 mV.
**b** Histogram of channel conductances obtained from 43 incorporation events. The black line depicts a Gaussian fit.
**c** Current - voltage dependence of the channel after incorporation.
**d** Typical current traces obtained from 'wildtype' channels (i-iii), and 'mutants' with modification M2 (iv-vi).
**e** Statistics of gating times for the wildtype (red) and the mutant (gray) channels. Continuous lines correspond to a mono-exponential fit for the wildtype channel ($\tau$=10.5 ms) and a double-exponential fit for the mutant ($\tau_1$=1.3 ms and $\tau_2$=9.2 ms).

**Figure 3** *DNA Translocation studies.*

**a** Addition of DNA hairpins (T5-HP-T50) to a DNA channel at V=200 mV results in the appearance of transient current blockades, indicating unzipping and translocation of hairpin molecules from cis to trans. When a sufficient amount of hairpins has accumulated on the trans side, they can also be transferred back from trans to cis by a reversal of the transmembrane voltage.
**b** Representative blockade events for forward (top) and backward (bottom) translocation of DNA hairpins.
**c** Scatter plot for the translocation of T5-HP-T50 DNA through a DNA channel from cis to trans (blue) and from trans to cis side (gray) at 200 mV bias potential, accompanied by the corresponding histograms. Each data point corresponds to a single translocation event. In total, 777 (forward) and 379 (backward) events were analyzed.

**d** Top: Typical current trace at 200 mV bias potential after addition of 10μM of Q-T60 DNA. Middle: Current trace after rinsing with buffer solution. Bottom: Current trace after subsequent addition of 10μM of Q-T125 DNA to the same channel.

**e** Representative blockade events for Q-T60 (top) and Q-T125 DNA (bottom).

**f** Scatter plot of the current blockade versus dwell time for the translocation of Q-T60 (red) and Q-T125 DNA (black) through the DNA channel. Each data point corresponds to a single translocation event. In total 631 (Q-T60) and 279 (Q-T125) events were analyzed. On the right and on the top corresponding histograms are shown. The lines correspond to single exponential (top) and Gaussian fits (right).

**Figure 4.** *Negative-stain TEM micrographs of synthetic DNA channels.*

**A:** Side view 1, **B:** Side view 2, rotated by 60° around the long axis relative to side view 1. Each micrograph of A and B is sized 110nm x 110nm. **C:** Top view. Each micrograph of C is sized 60nm x 60nm.
The first micrograph in the top row of each of the panels contains the respective average micrograph. The average micrographs are also depicted in Fig. 1d.

**Figure 5.** *Negative-stain TEM micrographs of cholesterol-modified DNA channels* attached to POPC SUs. Each micrograph is sized 60nm x 60nm. The first micrograph in the top row contains the respective average micrograph also depicted in Fig. 1e.

**Figure 6.** *Binding of cholesterol modified DNA channels to POPC membranes.*

**a** Cholesterol modified DNA channels mixed with SUVs and imaged byTEM. The lipids form white circular patches. Almost all DNA channels are attached to SUVs (collapsed on the TEM grid) or larger supported lipid membrane patches.

**b** Unmodified DNA channels mixed with SUVs show no binding - DNA channels and lipid patches typically are found isolated from each other.

Magnification 11500x (scale bars 250 nm).

**Figure 7.** *Binding of Cholesterol modified DNA channels to POPC membranes.*

**a** Cholesterol modified channels with SUVs,

**b** Unmodified channels with SUVs.

As in Fig.6 at a higher magnification (28500 x). Scale bar is 100 nm.

**Figure 8.** *Binding of DNA channels to DPhPC GUVs.*

**a.** Differential interference contrast images of GUVs in 300nMKClsolution.

**b.** Epifluorescence microscope images of YOYO labeled origami pores bound to the GUV membranes. The presence of labeled. DNA channels in solution leads to a strong fluorescence of the GUV membranes, suggesting that the DNA channels accumulate at the membrane surface due to binding interactions. A control experiment where only YOYO dye was used showed no observable fluorescence of the vesicle membrane. TEM images confirm that the intercalating dye YOYO does not alter the origami structure at the labeling density used here.

**Figure 9.** *Channel Incorporation*

**a** Top: Voltage pulse used for incorporation of origami channels into the lipid membrane. Bottom: Corresponding current response before (black dots) and during incorporation (red line). Inset: Consecutive incorporation of two origami channels at 100mV bias voltage.

**b** Noise power spectra before (black line) and after the incorporation of a single origami pore (red line). Current traces were recorded at 100mV at a filter frequency of 3kHz.

**Figure 10.** *Multiple insertions and stepwise closing.*

**a** Time course of the ionic current across the lipid bilayer with multiple origami channels incorporated at 200 mV bias voltage. The DNA channel structures used here had a 7nt modification at siteM2. Vertical lines mark limits of continuous recording. The recording was suspended a this positions with no applied bias for a few ms. Right: Corresponding all-point current histogram, showing a mean peak-to-peak distance of 172±15 pA This corresponds to a mean conductance change of 0.86±0.08 nS.
b Current response of origami channels at 200 mV with a modification present at site M1, accompanied by the corresponding all-point current histogram. The mean peak-to-peak distance corresponds to 194±14 pA (0.97±7nS).

**Figure *11.*** *FEMSimulations of the DNA pore.*

**a** Simulated conductance across a DNA channel incorporated into a lipid bilayer for varying intrinsic origami conductivity at 100mV bias voltage and 11S/m bulk solution conductivity.
**b** Color map of the electrical potential distribution along a y-z plane at x=0 calculated for 0.1 S/m origami conductivity. The dashed line marks the structure boundaries.
**c** Electric Field along the symmetry axis (z-axis) of the channel for varying origami conductivity.
**d** Corresponding potential drops along the channel.
**e** Schematic illustration of the penetration depth of the structures used in translocation experiments before unzipping (cf. Figure 3).

**Figure 12.** *Scatter plots of the relative conductance change versus dwell time for the closure states of unmodified origami channel compared to modified channels.*

**a** unmodified origami channel,
**b** a channel with modification on site M1 and
**c** a channel with modification on site M2
at 100mV bias voltage. Each data point corresponds to a single closing event. In total 697 events (red, a), 2532 events (blue, c) and 623 events (grey, b) were recorded.
On the right and on the top the corresponding histograms are shown. The lines correspond to single exponential (no modification or modification M2) or double exponential (with modification M1), (top) and Gaussian fits (right).
**d** Closure time for mutant M2 as a function of applied membrane voltage.

**Figure 13.** *Additional data for quadruplex translocations.*

**a** Scatter Plot of the current amplitude vs. dwell time for the backward translocation of Q-T125 through the artificial ion channel at negative bias voltage (-200mV), accompanied by the corresponding histograms. 266 events were detected. The solid lines correspond to single exponential (top) and Gaussian (right) fits to the histograms.
b Long-lived population: Scatter Plot of the current amplitude vs. dwell time for forward translocation of Q-T125 (see Fig. 3), including data from the last part of the measurement. Each data point corresponds to a single translocation event. The lines correspond to single exponential (top) and Gaussian (right) fits to the histograms.

EXAMPLES

**1. Materials and Methods**

**1.1 Assembly and purification of membrane channels**

*Molecular self assembly with scaffolded DNA origami.*

Structures were designed using caDNAno v.02 (Douglas et al., 2009-1). DNA scaffold strands of 7249 bases length derived from the genome of bacteriophage M13 (with SEQ ID NO. 1) were prepared recombinantly as previously described (Douglas et al., 2009-2). Staple oligonucleotide strands (with SEQ ID NOs. 2 to 131) were prepared by solid-phase chemical synthesis (Eurofins MWG, Ebersberg, Germany, HPSF purification). Production of DNA channel structures was accomplished in one-pot reactions by mixing 20 nM scaffold strands with 100 nM of oligonucleotide staple strands in a buffer including 5 mM TRIS, 1 mM EDTA, 20 mM $MgCl_2$, 5 mM NaCl (pH 8) (Douglas et al., 2009-2, Castro et al., 2011). The mixture was subjected to thermal annealing ramps that cooled from 65°C to room temperature. Shortly before purification the origami structures were incubated with 10μM of cholesterol-modified oligonucleotides at room temperature for one hour.

*Purification.*

50$\mu$l of the reaction products were mixed with 450$\mu$l of buffer containing 5 mMTris, 1 mM EDTA, 5 mM $MgCl_2$, 5 mM NaCl and spin filtered with a 100kDaAmicon filter (Millipore) at 2000rcf for 30 minutes. The filtration was repeated 5 additional times.

**1.2 Preparation of lipid membrane vesicles**

*Formation of small unilamellar vesicles (SUVs).*

POPC (1-palmitoyl-2-oleoyl-sn-glycero-3-phosphocholine) lipids were dissolved in chloroform to a concentration of 5 mg/ml. A lipid film was formed by evaporating 1 ml of POPC solution in a 5 ml round bottom flask, using a rotational evaporator. The flask was kept under vacuum for at least 30 minutes to evaporate remaining chloroform. The lipid film was resuspended in 2ml of a 150mM aqueous KCl solution. Subsequently, SUVs were formed by sonicating the solution with a tip sonicator (BandelinSonoplus mini20) for ca. 5 minutes at 8W, until the solution started to become transparent. Afterwards the SUVs were purified by centrifugation at 1500 rcf for 5 minutes. The solution was shock frozen with liquid nitrogen and kept at -80°C until usage.

*Preparation of giant unilamellar vesicles (GUVs).*

GUVs were created by electroswelling using the following procedure: 6$\mu$l (10mg/ml) DPhPC (1,2-diphytanoyl-sn-glycero-3-phosphocholine, Avanti Polar Lipids, Alabaster, AL, USA) with 10% Cholesterol were dissolved in Chloroform. The solution was dried on two parallel platinum electrodes, d=0.5mm, with a spacing of 2.5mm (center to center) for 1h under vacuum. The electrodes were then inserted into a 500$\mu$l eppendorf tube filled with 1.2 M sorbitol. The osmolarity was determined with an Osmomat 30 (Gonotec, Berlin). The osmolarity of the GUV buffer was adjusted by slightly changing the sorbitol concentration. Then a voltage of 3Vp-p at 5 Hz was applied for 3h.

**1.3 Imaging**

*Transmission election microscopy.*

Particles were adsorbed on glow-discharged formvar-supported carbon-coated Cu400 TEM grids (Science Services, Munich, Germany) and stained using a 2% aqueous uranylformate solution containing 25 mMNaOH. Imaging was performed using a Philips CM100 transmission electron microscope operated at 100 kV. Images were acquired using an AMT 4x4 Megapixel CCD camera. Micrograph scale bars were calibrated using 2D catalase crystal lattice constants as length reference. Imaging was performed at 28500x magnification for the majority of images and 11500x magnification for overview images.

*TEM image processing.*

Micrographs of individual particles were picked using the EMAN2 (Tang et al. 2007) boxer routine. Micrograph alignment and superposition was performed using the XMIPP mlf_2Dalign routine (Scheres et al., 2007).

*Fluorescence/DIC imaging of DNA channels on GUVs.*

$Mg^{2+}$ ions strongly influence the permeability of GUV membranes. In order to study interactions of DNA channels with GUVs, fluorescence imaging experiments were performed in a $Mg^{2+}$ free buffer containing 300mM KCl. Control TEM experiments confirmed the stability of DNA channels under these conditions. For staining, 4$\mu$l of DNA channel solution were incubated with 1$\mu$M (1$\mu$l) YOYO-1 (Invitrogen, Life Technologies GmbH, Darmstadt, Germany) for 2 hours in 600mMKCl, 0.5xTBE. Then, 5$\mu$l of the GUV solution were added and incubated for 1h. Next the GUV-Origami solution was diluted in 5 steps to a final concentration of 300mM KCl and 600mM sorbitol in order to reduce background fluorescence. (All other consumables were purchased form Sigma Aldrich, Munich, Germany). A microscopic observation chamber was assembled using vacuum grease and a cover glass, coated overnight with 5% BSA solution. For fluorescence and DIC imaging an Olympus 1X81 microscope with a 60x 1.4 oil immersion objective and a Hamamatsu Orca r2 camera was used.

### 1.4 Electrical measurements

All electrical experiments were performed using 16 channel microelectrode cavity arrays (MECA) (Baaken et al., 2008). The MECA chips were interfaced using an Orbit 16 device, provided by Nanion Technologies GmbH, Munich, Germany. Lipid solutions were prepared from 1,2-diphytanoyl-sn-glycero-3-phosphocholine (DPhPC, Avanti Polar Lipids, Alabaster, AL, USA) and dissolved in octane (Sigma Aldrich, Munich, Germany) at a concentration of 10 mg/ml. As electrolyte 1M KCl solution (Merck KGaA, Darmstadt, Germany) was used, containing 10mM Tris and 1 mM EDTA (Sigma Aldrich, Munich, Germany) at pH 8.

HPLC purified oligonucleotides for translocation studies (biomers, Ulm, Germany) were dissolved in 10 mMTris, 1mM EDTA solution at pH 8.
DNA sequences:

Hairpin: 5'- $(dT)_6$ - <u>ATCTACATA</u>TTTTTTTTTT<u>TATGTAGAT</u> - $(dT)_{50}$ -3' [SEQ ID NO. 133]
Q-T60: 5' - GGTTGGTGTGGTTGG - $(dT)_{60}$ -3' [SEQ ID NO. 134]
Q-T125: 5'- GGTTGGTGTGGTTGG - $(dT)_{12}$, -3' [SEQ ID NO. 135]

To form lipid bilayers on the 16-well MECA chips, 100 μl of electrolyte solution were added to the measurement chamber. In the MECA setup, the ground Ag/AgCl electrode resides in the *cis* (upper) compartment. 1-2 μl of lipid solution were pipetted in the vicinity of the cavities followed by painting the lipid bilayer on top of the cavities using a magnetic stir bar. Parallel current recordings were performed using a 16 Channel Triton+ amplifier (Tecella Inc., Costa Mesa, CA, USA) that was controlled via a custom made Labview (National Instruments Germany GmbH, Munich, Germany) measurement software. For low noise experiments on the gating behaviour of the DNA structures, individual channels were switched to a single channel patch clamp amplifier (EPC9, HEKA Elektronik GmbH, Lambrecht, Germany).

### 1.5 Data analysis

Patch clamp data were analyzed using custom routines implemented in Matlab (MathWorks, Ismaning, Germany). A cut-off frequency of 3 kHz was used for the built-in Bessel filter of the Tecella amplifier, but in fact the slow frequency response of the 16channel amplifier board limited the bandwidth considerably (0.5 - 1.7 kHz) in our experiments. To determine the amplitude of measured current blockades reliably, rising edge and falling edge of all peaks were fitted with single exponentials as described in (Baaken et al., 2008). All dwell time histograms feature logarithmically spaced bin widths and were fitted with a single exponential ($p(t)=\exp(-t/\tau)$) with the time constant $\tau$ being the only fit parameter. For measurements on hairpin translocation, it was necessary to introduce an amplitude scaling factor to obtain satisfying fits.

## 2. Results and Discussion

### 2.1 Quality control of DNA channels - TEM imaging

The quality of folding of each batch of DNA channels was assessed by transmission electron microscopy before the samples were used for electrical characterization. Typical results of this process are shown in Figure 4A-C.

### 2.2 Influence of cholesterol anchors on binding to lipid membranes

Binding to and insertion into lipid bilayer membranes is facilitated by cholesterol modifications of the DNA channels. To this end, the cap region of the channel structure facing the lipid membrane were modified at 26 positions arranged concentrically around the stem with single-stranded adaptor extensions (cf. schematic depiction in Fig. 1a, staple sequences SEQ ID NOs. 106-131 and caDNAno maps in 2.9). After folding of the channels, these extensions were hybridized to 22 nucleotide long cholesterol-modified DNA oligomers (an adaptor oligonucleotide) with sequence

5'-CCTCGCTCTGCTAATCCTGTTA-3' [SEQ ID NO. 132].

Depending on the position of the modification (5' or 3'), cholesterol can either be placed in close vicinity of the channel structure or within a distance of 22 bases from the cap. Both modification versions were investigated in our studies and showed electrically detectable interactions with the lipid membrane. The proximal modification version was found to lead to more stable insertions into the membrane, whereas the distal version mainly caused transient current fluctuations that were not stable upon voltage reversal.

As shown in Figures 5-7 and 8, we obtained electron and fluorescence microscopic evidence for binding of the channels to lipid bilayer membranes. DNA channels without cholesterol modification did not show any appreciable interaction with the membranes. We assume that upon insertion of the - negatively charged - DNA channels into the membrane, lipids rearrange around the stem of the channel to form a hydrophilic pore with lipid heads oriented toward the DNA stem. The cholesterol anchors prevent unbinding of the structure from membrane and stabilize the system.

### 2.3 Membrane insertion of DNA channels

For electrical measurements, a voltage pulse protocol was applied to facilitate the incorporation of single channels into the membrane. After the addition of origami channels to supported lipid membranes on the MECA chip, the voltage protocol shown in Fig. 9a is applied, supported by repeated mixing. After some time, typically a clear step in the current signal is observed along with an increase of 1/f noise (Fig. 9b). Typically this sharp increase in transmembrane current occurred during the initial part of the incorporation pulse (230mV for 20 ms), suggesting a voltage-enhanced incorporation probability as known for other biological channels.
Similar current steps could be observed at 100mV bias voltage, but less frequently. In some cases, two or more subsequent steps indicated the incorporation of multiple channels into the same membrane (see Fig. 9a inset). The flicker noise power varies greatly from channel to channel, from negligible up to values on the order of $10^{-6}$.

In experiments with elevated membrane voltages (e.g., constant V=200 mV), occasionally large conductances/currents were observed that corresponded to the presence of several channels in parallel (Fig. 10). In these cases, we observed stepwise closing of single channels over time, potentially caused by clogging of the channels. Partial recovery of the channel conductance is observed after setting the bias temporarily to 0 mV (Fig. 10a). As shown in Fig. 10, current histograms display the quantized conductance of the DNA channels.

### 2.4 Conductance of the DNA channels

A simple estimate for the conductance of the DNA channel is obtained by approximating it by a cylindrical resistor of equal dimensions (length L = 42 nm, diameter d = 2 nm) with additionalaccess resistances at the channel entrances. The conductance of such a cylinder is given by

$$G = \kappa \frac{\pi d^2}{4L + \pi d}$$

resulting in a conductance of 0.78 nS for a electrolytic conductivity of $\kappa$=10.86 S/m (for 1M KCl at T=25°C, Pratt et al., 2001). This result fits our experimental findings well. As observed previously (Wei et al., 2012), we also expected a considerable lateral conductivity, allowing ionic current also to flow from the sides of the origami cap or stem. Only in the membrane region the current is expected to be strictly confined to the central channel itself.

### 2.5 Mapping the potential inside the pore

A qualitative study of how the origami structure's intrinsic electrical conductivity impacts potential distribution and channel conductance was performed by means of a finite element simulation using Comsol Multiphysics to solve Poisson's equation. To this end the DNA channel was approximated by two cylinders with diameters 6.6 nm and 20 nm,with a cylindrical channel of diameter 2.2 nm inside (Fig. 11a). The lipid bilayer was modelled as a 4 nm thick layer with zero conductivity. The symmetry axis of the channel was defined as the z axis of the coordinate system. z=0 corresponds to the boundary of the lipid bilayer facing the stem side of the structure. The channel interior and adjacent solution compartments were simulated with KCl solution bulk conductivity (11 S/m). To screen the influence of origami leakiness, the conductivity of the origami structure was varied in the simulation from $10^{-4}$ S/m (insulating) to 10 S/m. The mesh density ranged from 18 elements/$nm^3$ (channel interior & surrounding) to $10^{-2}$ elements/$nm^3$ (solution compartments). Fig. 11 shows the results of the simulations. As expected, the calculated channel conductance coincides with that of a simple geometrical model for the limit of zero origami conductivity. For origami conductivity values of $10^{-1}$ S/m and more (corresponding to 1% of the bulk solution conductivity) a considerable increase in channel conductance is observed. In the bulk solution limit, the channel conductance corresponds to a channel with a length equal to the lipid bilayer thickness and a diameter equal to the stem's outer diameter. The electrical potential along the channel's central axis is shown in Fig. 11d. Even at comparably low origami conductivity, a considerable inhomogeneity of the potential drop is apparent. Notably, the region of steepest slope is not at the center of the channel structure, but at the bilayer position, i.e. 26 nm from the cis side channel entrance. The local variation of electric field strength for nonzero leak conductivity can be expected to strongly influence translocation experiments, where charged molecules are pulled electrophoretically through the pore (see 2.7 below).

### 2.6 Channel gating

As shown in Fig. 2, individual DNA channels displayed distinct gating behavior in which the channel conductance switched between several (sub)-conductance levels. While for high bias voltages the channels underwent full closure as seen, e.g., in Fig. 10, at lower voltages we typically observed current reduction amplitudes $\Delta I$ corresponding to fractions of a single channel conductance. We also designed channel 'mutants', in which one of the central helices forming the stem of the DNA channel was modified with a single-stranded heptanucleotide not incorporated into the main origami structure. This modification potentially could fluctuate, block the channel, or act as a 'handle' for the electric field and lead to partial unzipping of a DNA staple strand. We studied two channel mutants - for modification M1 the heptanucleotide pointed into the central channel, for modification M2 the staple extension was oriented away from the central stem (looking into one of the "other" holes of the cap).We found that both modifications increased the gating frequency and behaviour. Moreover, we never observed a current signal without gating for the mutants, whereas many of the unmodified DNA channels showed no gating at all (such as in current trace i) in Fig. 1d). Fig. 12a shows a scatter plot of conductance change vs. channel closure time for an unmodified DNA channel structure. A single population is observed with a mean conductance reduction of $\Delta G/\Delta G_0$ of 45% and an exponentially distributed dwell time with a mean value of 10.5 ms.As seen in Fig. 12b, for modification M1 (heptanucleotide pointing into the central channel) a clear second population occurs with a considerably higher blockade amplitude of 70% and a considerably shorter time constant of 0.3 ms.

Also for modification M2, a second population occurs. As expected for this mutant, the additional staple pointing away from the central stem does not lead to a stronger blockade amplitude - however, it causes gating fluctuations at short timescales similar to that of the A mutants.

We also studied the voltage dependence of gating fluctuations of the M2 mutant. Apparently, the closure time increases slightly with increasing voltage, indicating the coupling of the closure events to electrostatic forces. Notably, this clearly differentiates gating events from translocation events through the channel, which become faster with increasing voltage.

### 2.7 DNA translocation experiments

*Current blockade by DNA molecules with different tail lengths*

In our DNA translocation experiments, DNA molecules (hairpin and quadruplex structures) with polythymidine tails of different lengths (60 and 125 nt) were investigated for both forward (cis-trans) and backward (trans-cis) translocation directions (cf. Fig. lie). The fact that longer tails lead to a larger transient current reduction is another clear indication of translocation through the central channel of our DNA structures. Moreover, the current blockade amplitudes measured in these experiments can be used for a qualitative discussion of the electric field distribution within the DNA channel.

In a simple picture, the presence of a DNA strand of length $L_{DNA}$ reduces the effective diameter of the channel *from A* to $A\text{-}A_{DNA}$, where $A_{DNA}$ is the diameter of single-stranded DNA. Hence, the total conductance of the channel is given by the series of the conductances of blocked ($G_b$) and unblocked ($G_u$) part, i.e.,

$$G_b = \sigma \frac{A - A_{DNA}}{L_{DNA}}$$

$$G_u = \sigma \frac{A}{L - L_{DNA}}$$

$$G_{tot} = \frac{G_b G_u}{G_b + G_u} = \sigma \frac{A(A - A_{DNA})}{(A - A_{DNA})L + A_{DNA} L_{DNA}}$$

Compared with an unoccupied channel the relative current reduction (conductance reduction) is then given by:

$$\frac{\Delta I}{I} = \frac{\Delta G}{G} = 1 - \frac{(A - A_{DNA})L}{(A - A_{DNA})L + A_{DNA} L_{DNA}} = \frac{A_{DNA} L_{DNA}}{(A - A_{DNA})L + A_{DNA} L_{DNA}}$$

A 125 thymidine tail fully stretched to its contour length has a length of $L_{DNA} \approx 50$ nm, i.e., it spans the whole length of the DNA channel. Assuming an effective diameter of 1 nm for ssDNA, we would expect a current/conductance reduction of $\Delta G/G$ of $A_{DNA}/(A-A_{DNA}) \approx 33\%$ ($L=L_{DNA}$ in the formula above), which is considerably higher than what we observe (around 10%). A 60 nt long tail corresponds to a length of $L_{DNA} \approx 24$ nm (assuming a base-to-base distance of 0.4 nm), from which we expect a $\Delta G/G$ of $\approx 15\%$. While again our experimentally observed reduction is smaller (~ 5%), this value is completely consistent with the value for the 125 tail if one takes the leak conductance into account.

In Fig. 11e, the lengths of the DNA molecules together with the electric fields for different values of the leak conductivity are depicted. For finite leak conductivity, we expect a much stronger influence on the conductance from DNA molecules with long tails - traversing the high field region defined by the lipid bilayer membrane - than for the short tails. In fact, it is expected that short-tailed DNA molecules may feel a considerably smaller electric force depending on the exact field distribution. Furthermore, we expect short-tailed molecules to be sensitive to the translocation direction. As seen in the scatter plot Fig. 3c for DNA hairpins with a 50 nt tail, backward translocation results in faster translocations with a higher current amplitude, indicating that the short tail penetrates deeper into the high field region for the backward direction.

*Length of the DNA tails in the channel*

For translocation experiments we expect the DNA tails to be fully stretched in the channel, and therefore their lengths are simply given by the number of bases times the base-to-base-distance for ssDNA (0.4 nm - 0.5 nm). As the channel width (2 nm) is close to this length as well as to the persistence length of ssDNA, the usual theories for polymer conformation inside a narrow channel are not applicable - these refer either to the limiting case where the channel diameter is much larger than the persistence length (resulting in a scaling treatment) or much smaller than the persistence length (resulting in a mechanical deflection model) (Maarel 2007). A better molecular understanding of the conformation of DNA strands inside a DNA channel will require molecular modelling and simulation (Aksimentiev & Schulten, 2008).

In any case, for 100 mV applied bias over a length of L=42 nm, we expect electrostatic forces to be well in the higher pN regime (also supported by the fact that we observe force-induced melting of DNA duplexes in the translocation experiments). In free solution experiments, a single-stranded random coil structure would be completely stretched out at these forces, and we assume the same to be the case in the context of our translocation studies.

*Quadruplex translocation in backward direction*

In Fig. 13a, a scatter plot generated from backward translocation events (from trans to cis) of Q-T125 DNA at negative bias potential is shown. The scatter plot histograms yield a blockade amplitude that is 27% lower *(11.2 pA)* and a characteristic dwell time that is 2.3 times shorter (3.5 ms) than for the forward direction. This observation indicates a directionality of the translocation process itself, as the integrated electrophoretic force along the DNA channel should be independent of the direction of translocation (as the $T_{125}$ tail is expected to traverse the whole central channel). A possible reason for this effect may be steric or binding interactions at the entrance of the asymmetrically designed origami structure.

*Long-lived events for Q-T125 translocations at long measurement times*

For prolonged measurement times for the Q-T125 translocation experiment in forward direction (cf. Fig. 3 c)), a sudden increase in long-lived blockade events was observed. A scatter plot including all Q-T125 measurement data recorded for these events is shown in Figure 13b. In this plot, a second population is clearly visible. It features a higher blockade amplitude (20.5 pA) than the main population and a considerably longer characteristic dwell time (46 ms). A comparable population was not observed for the Q-T60 measurement or for the Q-T125 backward translocation measurement. One possible explanation for this time dependent effect is the agglomeration of DNA molecules at the channel entrance over the course of a measurement run. In fact, after addition of quadruplex DNA the event rate continuously increased, indicating that the DNA concentration at the pore entrance rises over time.

REFERENCES

Andersen, E. S. et al. Self-assembly of a nanoscale DNA box with a controllable lid. Nature 459, 73-76 (2009).

Akhshi P, Mosey NJ, Wu G. Free-energy landscapes of ion movement through a G-quadruplex DNA channel. Angew Chem Int Ed Engl. 51(12):2850-2854 (2012**).**

Aksimentiev, A. & Schulten, K. Imaging a-Hemolysin with Molecular Dynamics: Ionic Conductance, Osmotic Permeability, and the Electrostatic Potential Map. Biophys J88, 3745-3761 (2008).

Baaken, G., Sondermann, M., Schlemmer, C., Ruehe, J. & Behrends, J. C. Planar microelectrode-cavity array for high-resolution and parallel electrical recording of membrane ionic currents. Lab Chip 8, 938-944 (2008).
Bell, N. A. W. et al. DNA Origami Nanopores. Nano Lett 12, 512-517 (2012).
Boyer, P. D. (1997) Annu. Rev. Biochem. 66, 717-749
Branton, D. et al. The potential and challenges of nanopore sequencing. Nat. Biotech.26, 1146-1153 (2008).
Burge, S., Parkinson, G. N., Hazel, P., Todd, A. K. & Neidle, S. Quadruplex DNA: sequence, topology and structure. Nucleic Acids Res. (2006).
Castro, C. E. et al. A primer to scaffolded DNA origami. Nat Meth 8, 221-229 (2011).
Dietz, H., Douglas, S. M. & Shih, W. M. Folding DNA into Twisted and Curved Nanoscale Shapes. Science 325, 725-730 (2009).
Douglas, S. M. et al. Rapid prototyping of 3D DNA-origami shapes with caDNAno. Nucleic Acids Res. 37, 5001-5006 (2009).
Douglas, S. M. et al. Self-assembly of DNA into nanoscale three-dimensional shapes. Nature 459, 414-418 (2009).
Forman SL et al. Toward Artificial Ion Channels: A Lipophilic G-Quadruplex J. Am. Chem. Soc. 122, 4060-4067 (2000**)**.
Hall, A. R. et al. Hybrid pore formation by directed insertion of [alpha]-haemolysin into solid-state nanopores. Nat Nano 5, 874-877 (2010).
Han, D. et al. DNA origami with complex curvatures in three-dimensional space. Science 332, 342-346 (2011).
Hille, B. Ion channels of excitable membranes. 3rd edn, (Sinauer Associates, 2001).
Kaucher MS, Harrell WA Jr, Davis JT. A unimolecular G-quadruplex that functions as a synthetic transmembrane Na+ transporter. JAm Chem Soc. 128(1):38-39 (2006**)**.
Maarel, J. R. C. v. d. Introduction to Biopolymer Physics. (World Scientific, 2007).
Mathe, J., Visram, H., Viasnoff, V., Rabin, Y. & Meller, A. Nanopore Unzipping of Individual DNA Hairpin Molecules. Biophys. J. 87, 3205-3212 (2004).
Pratt, K., Koch, W., Wu, Y. & Berezansky, P. Molality-based primary standards of electrolytic conductivity - (IUPAC technical report). Pure Appl. Chem. 73, 1783-1793 (2001).
Rothemund, P. W. K. Folding DNA to create nanoscale shapes and patterns. Nature 440, 297-302 (2006).
Sakmann, B. & Neher, E. Single Channel Recording. (Plenum, 1995).
Scheres, S. H. W. et, al. Modeling experimental image formation for likelihood-based classification of electron microscopy. Structure 15, 1167-1177 (2007).
Seeman, N. C. Nanomaterials Based on DNA. Annu. Rev. Biochem. 79, 65-87 (2010).
Song, L. et al. Structure of staphylococcal alpha-hemolysin, a heptameric transmembrane pore. Science 274, 1859-1866 (1996).
Tang, G. et al. EMAN2: An extensible image processing suite for electron microscopy. J. Struct. Biol. 157, 38-46 (2007).
Wei, R., Martin, T. G., Rant, U. & Dietz, H. DNA Origami Gatekeepers for Solid-State Nanopores. Angewandte Chemie International Edition, 51(20):4864-7 (2012). Epub 2012 Apr 4.

## Claims

1. Nucleic acid based nanopore comprising one or more self-assembled nucleic acids and formed or assembled by the one or more nucleic acids, said self-assembled nucleic acids being obtained via molecular self-assembly of one or more scaffold nucleic acid strand(s) and/or a plurality of staple oligonucleotide strands,
comprising at least one recognition binding moiety,
wherein the nucleic acid based nanopore comprises

   (i) a transmembrane domain having a hollow channel and comprising a plurality of helical nucleic acid domains,
   (ii) optionally, a cap domain comprising helical nucleic acid domains,

   and wherein adhesion or attachment of the nanopore to a lipid bilayer, membrane or lipid vesicle via recognition binding moieties, leads to formation of a transmembrane channel which spans through said lipid bilayer, membrane or lipid vesicle.

2. The nucleic acid based nanopore of claim 1, wherein the at least one recognition binding moiety is attached to at least one of the nucleic acids,
wherein the recognition binding moiety is preferably

   - a hydrophobic moiety, preferably for binding to lipid or membrane surfaces, such as cholesterol or its derivatives, lipid(s), fatty acid(s), triglyceride(s),
   - a moiety specifically recognizing receptors or other surface molecules of (target) cell membranes,

such as
antibody or fragments thereof,
receptor ligand or fragments thereof, receptor fragments,
nucleic acid(s), such as aptamers,
antigen or epitopes.

**3.** The nucleic acid based nanopore of claim 1 or 2, wherein the nucleic acids comprise DNA, RNA, modified nucleic acids, artificial nucleic acids, nucleic acid analogues or combinations thereof,
and/or comprising self-assembled nucleic acids which are self-assembled DNA molecules, and/or wherein the transmembrane domain comprises parallel helical nucleic acid domains, preferably DNA double helices,
and/or wherein the cap domain is barrel-shaped, preferably comprising parallel DNA double helices.

**4.** The nucleic acid based nanopore of any of claims 1 to 3, wherein the hollow channel formed by the transmembrane domain has a length of about 2 to 200 nm,
and/or wherein the hollow channel formed by the transmembrane domain has a diameter of about 0.1 to 100 nm, preferably about 0.2 to 10 nm.

**5.** The nucleic acid based nanopore of any of the preceding claims, furthermore comprising at least one recognition sensing moiety, preferably attached to at least one of the nucleic acids, more preferably within the hollow channel of the transmembrane domain, wherein the recognition sensing moiety is preferably a moiety specifically recognizing or binding an analyte or molecule which passes through the nanopore or hollow channel.

**6.** The nucleic acid based nanopore of any of the preceding claims, comprising one or more further components, such as

- label or tag,
such as fluorescent dye, chromophore, isotope, quantum dot,
- protein or peptide,
such as an enzyme or its binding and/or catalytic domain, antibody or fragments thereof, receptor ligand or fragments thereof, receptor fragments, antigen or epitopes, preferably to be delivered to a target cell,
- drug,

such as a drug to be delivered to a target cell,
such as antimicrobial or anti-viral drugs, anti-cancer drugs,

- nanoparticle or nanocrystal,
- glycosylated or glycosylation label or tag,
such as glycan, glycoprotein, glycolipid, proteoglycan,
- component for a functional mechanism,

such as
a rotor domain enabling the nanopore to move or rotate in the lipid bilayer or membrane,
an ion pump,
transport proteins or domains thereof,

preferably attached to at least one of the nucleic acids.

**7.** The nucleic acid based nanopore of any of the preceding claims, comprising gating mechanism component(s) for opening and closing the nanopore,
such as gating mechanism component(s) that are reactive to binding of ligand(s), such as antigen(s), pressure, temperature, voltage and/or light.

**8.** The nucleic acid based nanopore of any of the preceding claims, wherein the recognition binding moiety(ies), the recognition sensing moiety(ies), the further component(s) and/or the gating mechanism component(s) comprise adaptor oligonucleotide(s) with a nucleotide sequence which hybridizes to at least one of the nucleic acids.

**9.** The nucleic acid based nanopore of any of the preceding claims, wherein the self-assembled nucleic acids are obtained
via DNA origami molecular self-assembly of scaffold nucleic acid strand(s) and a plurality of staple oligonucleotide strands, or
via molecular self-assembly of one or more scaffold nucleic acid strand(s), or
via molecular self-assembly of a plurality of oligonucleotide strands.

**10.** The nucleic acid based nanopore of claim 9, wherein the scaffold nucleic acid strand is a single stranded polynucleotide strand, which is derived from a natural source, such as derived from M13 phage DNA (such as a nucleic acid comprising the nucleotide sequence of SEQ ID NO. 1), or is artificial;
and/or wherein the staple oligonucleotide strands are at least partially complementary to parts of the scaffold nucleic acid strand,
and are preferably oligonucleotides comprising a nucleotide sequence of SEQ ID NOs. 2 to 131;
and/or wherein the adaptor oligonucleotides comprised in the recognition binding moiety(ies), the recognition sensing moiety(ies), the further component(s) and/or the gating mechanism component(s) are selected from oligonucleotides comprising a nucleotide sequence of SEQ ID NO. 132.

**11.** The nucleic acid based nanopore of any of the preceding claims, comprising a modification in the hollow channel of the transmembrane domain, such as single stranded nucleic acids or oligonucleotides, preferably for varying the conductivity of the nanopore.

**12.** Nanopore complex comprising at least one nucleic acid based nanopore of any one of claims 1 to 11, preferably comprising two or more of said nucleic acid based nanopores, such as nucleic acid based nanopores which differ in their recognition moieties, further component(s) and/or gating mechanisms.

**13.** Use of a nucleic acid based nanopore of any one of claims 1 to 11 or the nanopore complex of claim 12 as sensor, preferably single molecule sensor; and/or for controlling membrane potentials, transmembrane traffic and/or interference with membrane fucnctions.

**14.** Use of a nucleic acid based nanopore of any one of claims 1 to 11 or the nanopore complex of claim 12 and the use according to claim 13, comprising

- nucleic acid sequencing,
- sensing nucleic acid secondary structure,
- detection of analytes, such as small molecules, toxins, proteins,
- detection of polymers,
- separation of components across a lipid bilayer or a (cell) membrane,
- separation of charges across a lipid bilayer or a (cell) membrane,
- molecular filters and sieves,

preferably comprising voltage gating, pressure, gating, ligand-gating, or gating by other physical effects, such as light, temperature.

**15.** The nucleic acid based nanopore of any one of claims 1 to 11 or the nanopore complex of claim 12 for the diagnosis of a disease, such as cancer, infectious diseases, allergies, metabolic diseases, obesity and heart diseases, preferably comprising the cell-specific targeting of diseased cells, tissues and organs; cell-specific labelling of diseased cells, tissues and organs.

**16.** Composition for obtaining a nucleic acid based nanopore of any one of claims 1 to 11 comprising
a scaffold nucleic acid strand derived from M13 phage DNA being a nucleic acid comprising the nucleotide sequence of SEQ ID NO. 1 and/or a plurality of staple oligonucleotide strands which are at least partially complementary to parts of the scaffold nucleic acid strand and are oligonucleotides comprising a nucleotide sequence of SEQ ID NOs. 2 to 131 as defined in claim 12,
adaptor oligonucleotides comprising recognition binding moiety(ies) as defined in claim 10 and 12,
optionally, adaptor oligonucleotides comprising recognition sensing moiety(ies) as defined in claim 10 and 12,
optionally, oligonucleotides comprising further component(s) and/or gating mechanism component(s) as defined in claim 10 and 12,
wherein the adaptor oligonucleotides comprised in the recognition binding moiety(ies), the recognition sensing moiety(ies), the further component(s) and/or the gating mechanism component(s) are selected from oligonucleotides comprising a nucleotide sequence of SEQ ID NO. 132,
suitable buffers and salts.

## Patentansprüche

1. Nulcleinsäure-basierende Nanopore umfassend eine oder mehrere selbst-assemblierte Nukleinsäuren und gebildet oder assembliert durch die eine oder mehreren Nukleinsäuren, wobei die selbst-assemblierten Nukleinsäuren durch molekulare Selbst-Assemblierung von einem oder mehreren Gerüst-Nukleinsäure-Strängen und/oder einer Vielzahl von Stapel-Oligonukleotid-Strängen erhalten wird,
umfassend mindestens einen Erkennungs-Bindungsrest,
wobei die Nukleinsäure-basierende Nanopore

    (i) eine Transmembran-Domäne, die einen hohlen Kanal aufweist und eine Vielzahl an helikalen Nukleinsäure-Domänen umfasst,
    (ii) optional, eine Kappen-Domäne umfassend helikale Nukleinsäure- Domänen, umfasst,

    und wobei Adhäsion oder Anlagerung der Nanopore an eine Lipid-Doppelschicht, eine Membran oder ein Lipid-Vesikel über Erkennungs-Bindungsreste zur Bildung eines Transmembran-Kanals führt, der sich durch die Lipid-Doppelschicht, die Membran oder das Lipid-Vesikel erstreckt.

2. Die Nukleinsäure-basierende Nanopore von Anspruch 1, wobei der mindestens eine Erkennungs-Bindungsrest an mindestens eine der Nukleinsäuren angehängt ist,
wobei der Erkennungs-Bindungsrest bevorzugt

    - ein hydrophober Rest, bevorzugt für die Bindung an Lipid- oder Membranoberflächen,
    wie beispielsweise Cholesterol oder dessen Derivative, Lipid(e), Fettsäure(n), Triglycerid(e),
    - ein Rest, der spezifisch Rezeptoren oder andere Oberflächenmoleküle der (Ziel-) Zellmembranen erkennt,

        wie beispielsweise
        Antikörper oder Fragmente davon,
        Rezeptorligand oder Fragmente davon, Rezeptor-Fragmente, Nukleinsäure(n), wie Aptamere,
        Entigen oder Epitope,

    ist.

3. Die Nukleinsäure-basierende Nanopore von Anspruch 1 oder 2, wobei die Nukleinsäuren DNA, RNA, modifizierte Nukleinsäuren, künstliche Nukleinsäuren, Nukleinsäure-Analoga oder Kombinationen davon umfassen,
und/oder umfassend selbst-assemblierte Nukleinsäuren, die selbst-assemblierte DNA-Moleküle sind,
und/oder wobei die Transmembran-Domäne parallel helikale Nukleinsäure-Domänen, bevorzugt DNA-Doppelhelices, umfasst;
und/oder wobei die Kappen-Domäne fassförmig ist, bevorzugt umfassend parallele DNA-Doppelhelices.

4. Die Nukleinsäure-basierende Nanopore gemäß einem der Ansprüche 1 bis 3, wobei der hohle Kanal gebildet von der Transmembran-Domäne eine Länge von ungefähr 2 bis 200 nm aufweist,
und/oder wobei der hohle Kanal gebildet von der Transmembran-Domäne einen Durchmesser von ungefähr 0,1 bis 100 nm, bevorzugt ungefähr 0,2 bis 10 nm, aufweist.

5. Die Nukleinsäure-basierende Nanopore gemäß einem der vorangehenden Ansprüche, weiterhin umfassend mindestens einen Erkennungs-Sensor-Rest, bevorzugt angehängt an mindestens eine der Nukleinsäuren, weiter bevorzugt innerhalb des hohlen Kanals der Transmembran-Domäne,
wobei der Erkennungs-Sensor-Rest bevorzugt ein Rest ist, der spezifisch einen Analyt oder ein Molekül erkennt oder bindet, das durch die Nanopore oder den hohlen Kanal hindurch geht.

6. Die Nukleinsäure-basierende Nanopore gemäß einem der vorangehenden Ansprüche, umfassend eine oder mehrere weitere Komponenten, wie beispielsweise

    - eine Markierung oder ein Tag,
    wie beispielsweise fluoreszierender Farbstoff, Chromophor, Isotop, Quantenpunkt,
    - Protein oder Peptid,
    wie beispielsweise ein Enzym oder seine Bindungs- und/oder katalytische Domäne, Antikörper oder Fragmente davon, Rezeptorligand oder Fragmente davon, Rezeptorfragmente, Antigen oder Epitope, welche(s) bevorzugt an eine Zielzelle geliefert werden soll(en),
- Wirkstoff,
wie beispielsweise ein Wirkstoff, der an eine Zielzelle geliefert werden soll, wie beispielsweise antimikrobielle oder anti-virale Wirkstoffe, Anti-Krebswirkstoffe,
- Nanopartikel oder Nanokristall,
- glykosylierte oder Glykosylierungs-Markierung oder Tag,
wie beispielsweise Glylcan, Glylcoprotein, Glykolipid, Proteoglycan,
- Komponente für einen funktionalen Mechanismus,

wie beispielsweise
eine Rotordomäne, die die Nanopore befähigt, sich in der Lipid-Doppelschicht oder Membran zu bewegen oder zu rotieren,
eine Ionenpumpe,
Transportproteine oder Domänen davon,

bevorzugt angehängt an mindestens eine der Nukleinsäuren.

**7.** Die Nukleinsäure-basierende Nanopore gemäß einem der vorangehenden Ansprüche, umfassend Komponente(n) eines Tormechanismus für das Öffnen und Schließen der Nanopore,
wie beispielsweise Komponente(n) eines Tormechanismus, die reaktiv auf die Bindung von Ligand(en) isr/sind, wie beispielsweise Antigen(e), Druck, Temperatur, Spannung und/oder Licht.

**8.** Die Nukleinsäure-basierende Nanopore gemäß einem der vorangehenden Ansprüche, wobei der/die Erkennungs-Bindungsrest(e), der/die Erkennungs-Sensor-Rest(e), die weitere(n) Komponente(n) und/oder die Komponente(n) eines Tormechanismus Adaptor-Oligonukleotid(e) mit einer Nukleotidsequenz umfassen, die an mindestens eine der Nukleinsäure hybridisiert.

**9.** Die Nukleinsäure-basierende Nanopore gemäß einem der vorangehenden Ansprüche, wobei die selbst-assemblierten Nukleinsäuren erhalten werden
via DNA-Origami molekulare Selbst-Assemblierung von Gerüst-Nukleinsäure-Strängen und/oder einer Vielzahl von Stapel-Oligonukleotid-Strängen, oder
via molekulare Selbst-Assemblierung von einem oder mehreren Gerüst-Nukleinsäure-Strängen, oder
via molekulare Selbst-Assemblierung einer Vielzahl von Stapel-Oligonukleotid-Strängen.

**10.** Die Nukleinsäure-basierende Nanopore gemäß Anspruch 9, wobei der Gerüst-Nukleinsäure-Strang ein einzelsträngiger Polynukleotidstrang ist, der von einer natürlichen Quelle abgeleitet wurde, wie beispielsweise abgeleitet von M13 Phagen-DNA (wie beispielsweise eine Nukleinsäure umfassend die Nukleotidsequenz von SEQ ID NO. 1), oder künstlich ist;
und/oder wobei die Stapel-Oligonukleotid-Stränge mindestens partiell komplementär zu Teilen des Gerüst-Nukleinsäure-Strangs sind,
und bevorzugt Oligonukleotiden sind, die eine Nukleotidsequenz der SEQ ID NOs. 2 bis 131 umfassen;
und/oder wobei die Adaptor-Oligonukleotide umfasst in dem/den Erkennungs-Bindungsrest(en), dem/den Erkennungs-Sensor-Rest(en), der/den weiteren Komponente(n) und/oder der/den Komponente(n) eines Tormechanismus ausgewählt sind aus Oligonukleotiden umfassend eine Nukleotidsequenz von SEQ ID NO. 132.

**11.** Die Nukleinsäure-basierende Nanopore gemäß einem der vorangehenden Ansprüche, umfassend eine Modifizierung im hohlen Kanal der Transmembran-Domäne, wie beispielsweise einzelsträngige Nukleinsäuren oder Oligonukleotide, bevorzugt um die Konduktivität der Nanopore zu variieren.

**12.** Nanoporen-Komplex umfassend mindestens eine Nukleinsäure-basierende Nanopore gemäß einem der Ansprüche 1 bis 11, bevorzugt umfassend zwei oder mehrere der Nukleinsäure-basierenden Nanoporen, wie beispielsweise Nukleinsäure-basierende Nanoporen, die sich in ihren Erkennungsresten, weiterer/n Komponente(n) und oder Tormechanismus unterscheiden.

**13.** Verwendung einer Nukleinsäure-basierenden Nanopore gemäß einem der Ansprüche 1 bis 11 oder des Nanoporen-Komplex gemäß Anspruch 12 als Sensor, bevorzugt Einzelmolekül-Sensor;
und/oder für die Kontrolle von Membranpotentialen, Transmembranverkehr und/oder Interferenz mit Membranfunktionen.

**14.** Verwendung einer Nukleinsäure-basierenden Nanopore gemäß einem der Ansprüche 1 bis 11 oder des Nanoporen-Komplex gemäß Anspruch 12 und die Verwendung gemäß Anspruch 13, umfassend

- Nukleinsäure-Sequenzierung,
- Wahrnehmen der Sekundärstruktur von Nukleinsäuren,
- Detektion von Analyten, wie beispielsweise kleine Moleküle, Toxine, Proteine,
- Detektion von Polymeren,
- Separation von Komponenten durch eine Lipid-Doppelschicht oder eine (Zell-) Membran,
- Separation von Ladungen durch eine Lipid-Doppelschicht oder eine (Zell-)Membran,
- molekulare Filter und Siebe,

bevorzugt umfassend Spannungstakten, Druck, Takten ("gating"), Ligand-Takten, oder Takten durch andere physikalische Effekte, wie beispielsweise Licht, Temperatur.

**15.** Die Nukleinsäure-basierende Nanopore gemäß einem der Ansprüche 1 bis 11 oder der Nanoporen-Komplex gemäß Anspruch 12 für die Diagnose einer Erkrankung, wie beispielsweise Krebs, Infektionserkrankungen, Allergien, metabolische Erkrankungen, Obesitas und Herzerkrankungen,
bevorzugt umfassend das zell-spezifische Abzielen auf erkrankte Zellen, Gewebe und Organe; das zell-spezifische Markieren von erkrankten Zellen, Geweben und Organen.

**16.** Zusammensetzung zum Erhalten einer Nukleinsäure-basierenden Nanopore gemäß einem der Ansprüche 1 bis 11, umfassend
einen Gerüst-Nukleinsäure-Strang abgeleitet von M13 Phagen-DNA, welches eine Nukleinsäure- ist umfassend die Nukleotidsequenz von SEQ ID NO. 1 und/oder eine Vielzahl von Stapel-Oligonukleotid-Strängen, die mindestens partiell komplementär zu Teilen des Gerüst-Nukleinsäure-Strangs sind und Oligonukleotide sind umfassend eine Nukleotidsequenz von SEQ ID NOs. 2 bis 131, wie definiert in Anspruch 12, Adaptor-Oligonukleotide umfassend Erkennungs-Bindungsrest(e), wie definiert in Anspruch 10 und 12,
optional, Adaptor-Oligonukleotide umfassend Erkennungs-Sensorrest(e), wie definiert in Anspruch 10 und 12,
optional, Oligonukleotide umfassend weitere Komponente(n) und/oder Komponente(n) eines Tormechanismus, wie definiert in Anspruch 10 und 12,
wobei die Adaptor-Oligonukleotide umfasst in dem/den Erkennungs-Bindungsrest(en), dem/den Erkennungs-Sensorrest(en), der/den weiteren Komponente(n) und/oder der/den Komponente(n) eines Tormechanismus ausgewählt sind aus Oligonukleotiden umfassend eine Nukleotidsequenz von SEQ ID NO. 132, geeignete Puffer und Salze.

## Revendications

**1.** Nanopore à base d'acide nucléique comprenant un ou plusieurs acides nucléiques auto-assemblés, et formés ou assemblés par l'un ou plusieurs acides nucléiques, lesdits acides nucléiques auto-assemblés étant obtenus par l'intermédiaire d'un auto-assemblage moléculaire d'un ou de plusieurs brin(s) d'acide nucléique en échafaudage et/ou d'une pluralité de brins d'oligonucléotide de type agrafe,
comprenant au moins un groupement de liaison de reconnaissance,
où le nanopore à base d'acide nucléique comprend

(i) un domaine transmembranaire ayant un canal creux et comprenant une pluralité de domaines d'acide nucléique en hélice,
(ii) éventuellement, un domaine de coiffe comprenant des domaines d'acides nucléiques en hélice, et où l'adhésion ou la fixation du nanopore à une bicouche de lipide, à une membrane ou à une vésicule de lipide par l'intermédiaire des groupements de liaison de reconnaissance mène à la formation d'un canal transmembranaire qui enjambe la bicouche de lipide, la membrane ou la vésicule de lipide.

**2.** Nanopore à base d'acide nucléique selon la revendication 1, où l'au moins un groupement de liaison de reconnaissance est attaché à au moins l'un des acides nucléiques, où le groupement de liaison de reconnaissance est de préférence

- un groupement hydrophobe, de préférence, destiné à se lier à des surfaces de lipides ou de membranes, telles que le cholestérol ou ses dérivés, un(des) lipide(s), un(des) acide(s) gras, un(des) triglycéride(s),
- un groupement reconnaissant de manière spécifique des récepteurs ou d'autres molécules de surface de membranes cellulaires (cibles),
tels
qu'un anticorps ou des fragments de celui-ci, qu'un ligand récepteur ou des fragments de celui-ci, des fragments de récepteur,
qu'un(que des) acide(s) nucléique(s), tel(s) qu'un(que des) aptamère(s),
qu'un antigène ou des épitopes.

**3.** Nanopore à base d'acide nucléique selon la revendication 1 ou 2, où les acides nucléiques comprennent de l'ADN, de l'ARN, des acides nucléiques modifiés, des acides nucléiques artificiels, des analogues d'acides nucléiques ou des combinaisons de ceux-ci,
et/ou comprenant des acides nucléiques auto-assemblés qui sont des molécules d'ADN auto-assemblées,
et/ou où le domaine transmembranaire comprend des domaines d'acide nucléique en hélice parallèles, de préférence, des hélices doubles d'ADN,
et/ou où le domaine de coiffe est en forme de tonneau, comprenant de préférence des hélices doubles d' ADN.

**4.** Nanopore à base d'acide nucléique selon l'une quelconque des revendications 1 à 3, où le canal creux formé par le domaine transmembranaire a une longueur d'environ 2 à 200 nm,
et/ou le canal creux formé par le domaine transmembranaire a un diamètre d'environ 0,1 à 100 nm, de préférence, d'environ 0,2 à 10 nm.

**5.** Nanopore à base d'acide nucléique selon l'une quelconque des revendications précédentes, comprenant en outre au moins un groupement de détection de reconnaissance, de préférence, attaché à au moins l'un des acides nucléiques, plus préférentiellement, dans le canal creux du domaine transmembranaire, où le groupement de détection de reconnaissance est de préférence un groupement spécifiquement de reconnaissance ou de liaison d'un analyte ou d'une molécule qui passe à travers le nanopore ou le canal creux.

**6.** Nanopore à base d'acide nucléique selon l'une quelconque des revendications précédentes, comprenant un ou plusieurs autres composants, tels

- qu'une étiquette ou un marquage,
tel qu'un colorant fluorescent, un chromophore, un isotope, un point quantique,
- qu'une protéine ou un peptide,
tel qu'une enzyme ou son domaine de liaison et/ou catalytique, un anticorps ou des fragments de celui-ci, un ligand de récepteur ou des fragments de celui-ci, des fragments de récepteur, un antigène ou des épitopes, de préférence devant être délivrés à une cellule cible,
- qu'un médicament,
tel qu'un médicament devant être délivré à une cellule cible,
tel que des médicaments antimicrobiens ou antiviraux, des médicaments anti-cancers,
- qu'une nanoparticule ou un nanocristal,
- qu'une étiquette ou un marquage glycosylé ou de glycosylation,
tel qu'un glycane, une glycoprotéine, un glycolipide, un protéoglycane,
- qu'un composant pour un mécanisme fonctionnel, tel que
un domaine rotor permettant au nanopore de se déplacer ou de se mettre en rotation dans la bicouche de lipide ou membrane,
une pompe ionique,
des protéines de transport ou des domaines de celles-ci,
de préférence attaché à au moins l'un des acides nucléiques.

**7.** Nanopore à base d'acide nucléique selon l'une quelconque des revendications précédentes, comprenant un(des)composant(s) de mécanisme de déclenchement qui sont réactifs à la liaison d'un(de) ligand(s), tels qu'un(que des) antigène(s), à la pression, à la température, à la tension et/ou à la lumière.

**8.** Nanopore à base d'acide nucléique selon l'une quelconque des revendications précédentes, où le(les) groupement(s) de liaison de reconnaissance, le(les) groupement(s) de détection de reconnaissance, l'autre(les autres) composant(s) et/ou composant(s) de mécanisme de déclenchement comprennent un(des) adaptateur(s) oligonucléotide(s) avec une séquence de nucléotides qui s'hybridise à au moins l'un des acides nucléiques.

**9.** Nanopore à base d'acide nucléique selon l'une quelconque des revendications précédentes, où les acides nucléiques auto-assemblés sont obtenus
par un auto-assemblage moléculaire d'origami d'ADN d'un(de) brin(s) d'acide nucléique en échafaudage et une pluralité de brins d'oligonucléotide de type agrafe, ou
par un auto-assemblage moléculaire d'un ou de plusieurs brin(s) d'acide nucléique en échafaudage, ou
par un auto-assemblage moléculaire d'une pluralité de brins d'oligonucléotide.

**10.** Nanopore à base d'acide nucléique selon la revendication 9, où le brin d'acide nucléique en échafaudage est un brin de polynucléotide à simple brin qui est dérivé d'une source naturelle, tel que dérivé d'ADN de phage M13 (tel qu'un acide nucléique comprenant une séquence de nucléotide de la SEQ ID N °1), ou est artificiel ;
et/ou où les brins d'oligonucléotide de type agrafe sont au moins partiellement complémentaires à des parties du brin d'acide nucléique en échafaudage,
et sont de préférence des oligonucléotides comprenant une séquence de nucléotide des séquences SEQ ID N °2 à 131 ;
et/ou où les adaptateurs d'oligonucléotides compris dans le(les) groupement(s) de liaison de reconnaissance, le (les) groupement(s) de détection de reconnaissance, l'autre(les autres) composant(s) et/ou le(les) composant(s) de mécanisme de déclenchement sont choisis parmi des oligonucléotides comprenant une séquence de nucléotide de la SEQ ID N ° 132.

**11.** Nanopore à base d'acide nucléique selon l'une quelconque des revendications précédentes, comprenant une modification dans le canal creux du domaine transmembranaire, comme des acides nucléiques ou des oligonucléotides à simple brin, de préférence, pour varier la conductivité du nanopore.

**12.** Complexe nanopore comprenant au moins un nanopore à base d'acide nucléique selon l'une quelconque des revendications 1 à 11, de préférence, comprenant deux ou plusieurs desdits nanopores à base d'acide nucléique, tels que des nanopores à base d'acide nucléique qui diffèrent dans leurs groupements de reconnaissance, d'un autre(d'autres) composant(s) et/ou de mécanismes de déclenchement.

**13.** Utilisation d'un nanopore à base d'acide nucléique selon l'une quelconque des revendications 1 à 11 ou du complexe nanopore selon la revendication 12 en tant que capteur, de préférence, de capteur de molécule unique ;
et/ou pour contrôler des potentiels de membranes, un trafic transmembranaire et/ou une interférence avec des fonctions de membranes.

**14.** Utilisation d'un nanopore à base d'acide nucléique selon l'une quelconque des revendications 1 à 11 ou d'un complexe nanopore selon la revendication 12, et l'utilisation selon la revendication 13, comprenant

- un séquençage d'acide nucléique,
- une perception d'une structure secondaire d'acide nucléique,
- une détection d'analytes, tels que des petites molécules, des toxines, des protéines,
- une détection de polymères,
- une séparation de composants à travers une bicouche de lipide ou une membrane (cellulaire),
- une séparation de charges à travers une bicouche de lipide ou une membrane (cellulaire),
- des filtres et des tamis moléculaires,

de préférence, comprenant un déclenchement de tension, une pression, un déclenchement, un déclenchement de ligand, ou un déclenchement par d'autres effets physiques, tels que la lumière, la température.

**15.** Nanopore à base d'acide nucléique selon l'une quelconque des revendications 1 à 11, ou le complexe nanopore selon la revendication 12, pour le diagnostic d'une maladie, telle qu'un cancer, de maladies infectieuses, d'allergies, de maladies métaboliques, de l'obésité et de maladies cardiaques,
comprenant de préférence le ciblage spécifique à la cellule de cellules malades, de tissus et d'organes ; le marquage spécifique à la cellule de cellules malades, de tissus et d'organes.

**16.** Composition pour obtenir un nanopore à base d'acide nucléique selon l'une quelconque des revendications 1 à 11 comprenant
un brin d'acide nucléique en échafaudage dérivé d'ADN de phage M13 étant un acide nucléique comprenant la séquence de nucléotide de la SEQ ID N °1 et/ou une pluralité de brins d'oligonucléotide de type agrafe qui sont au moins partiellement complémentaires à des parties du brin d'acide nucléique en échafaudage et sont des oligonucléotides comprenant une séquence de nucléotide des séquences SEQ ID N ° 2 à 131 telles que définies en revendication 12,
un adaptateur d'oligonucléotides comprenant un (des) groupement(s) de liaison de reconnaissance tels que définis dans les revendications 10 et 12,
éventuellement, un adaptateur d'oligonucléotides comprenant un(des) groupement(s) de détection de reconnaissance tels que définis en revendications 10 et 12,
éventuellement, des oligonucléotides comprenant un autre(d'autres) composant(s) et/ou un(des) composant(s) de mécanisme de déclenchement tels que définis dans les revendications 10 et 12,
où l'adaptateur d'oligonucléotides compris dans le(les) groupement(s) de liaison de reconnaissance, le (les) groupement(s) de détection de reconnaissance, l'autre(les autres) composant(s) et/ou le(les) composant(s) de mécanisme de déclenchement sont choisis parmi des oligonucléotides comprenant une séquence de nucléotide selon la SEQ ID N °132, des tampons et des sels appropriés.

# Figure 1

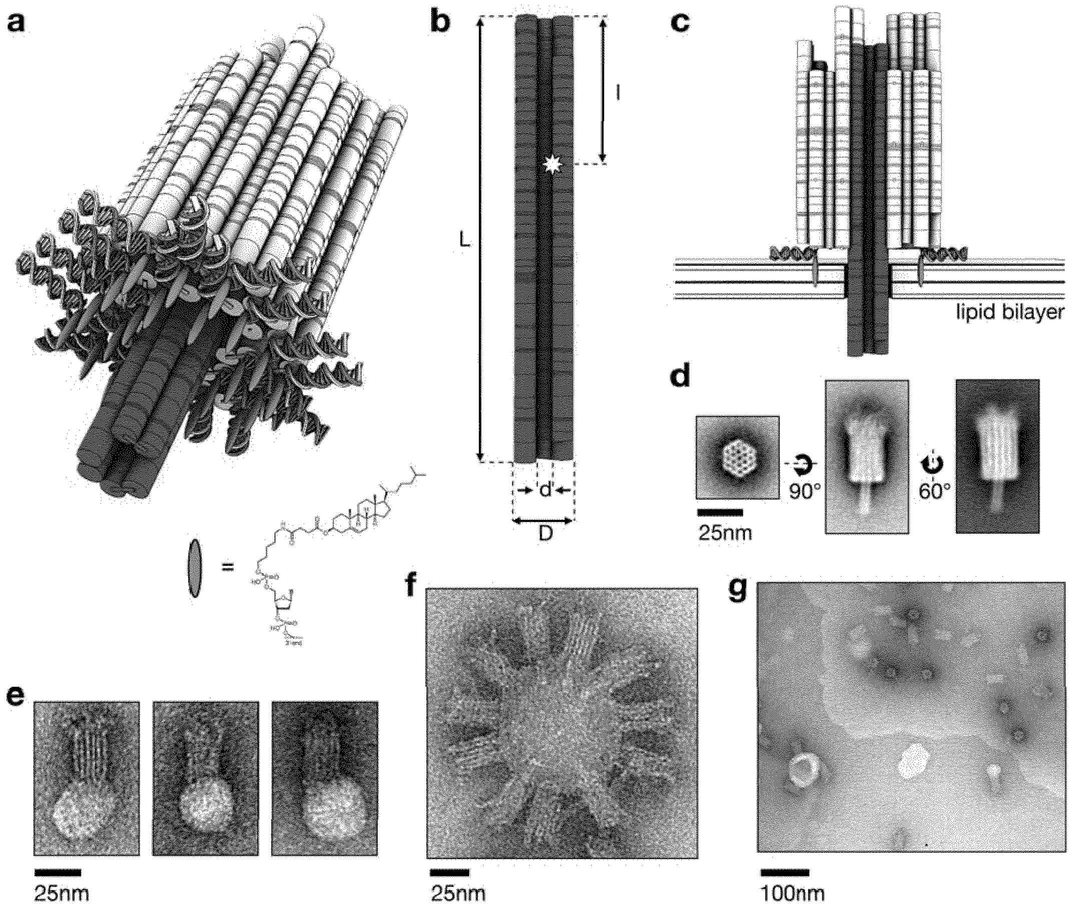

# Figure 2

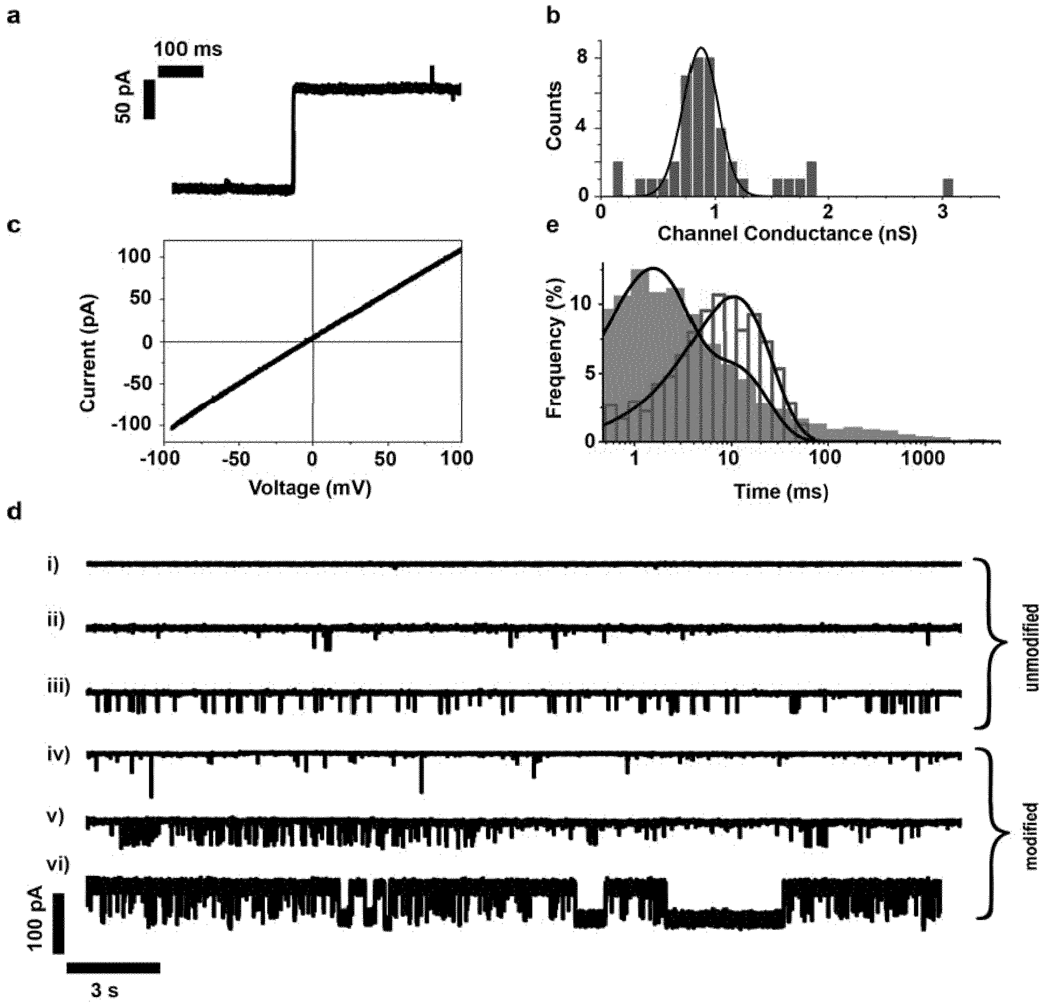
a
100 ms
50 pA
b
Counts
8
4
0
0
1
2
3
Channel Conductance (nS)
c
Current (pA)
100
50
0
-50
-100
-100
-50
0
50
100
Voltage (mV)
e
Frequency (%)
10
5
0
1
10
100
1000
Time (ms)
d
i)
ii)
iii)
iv)
v)
vi)
unmodified
modified
100 pA
3 s

# Figure 3

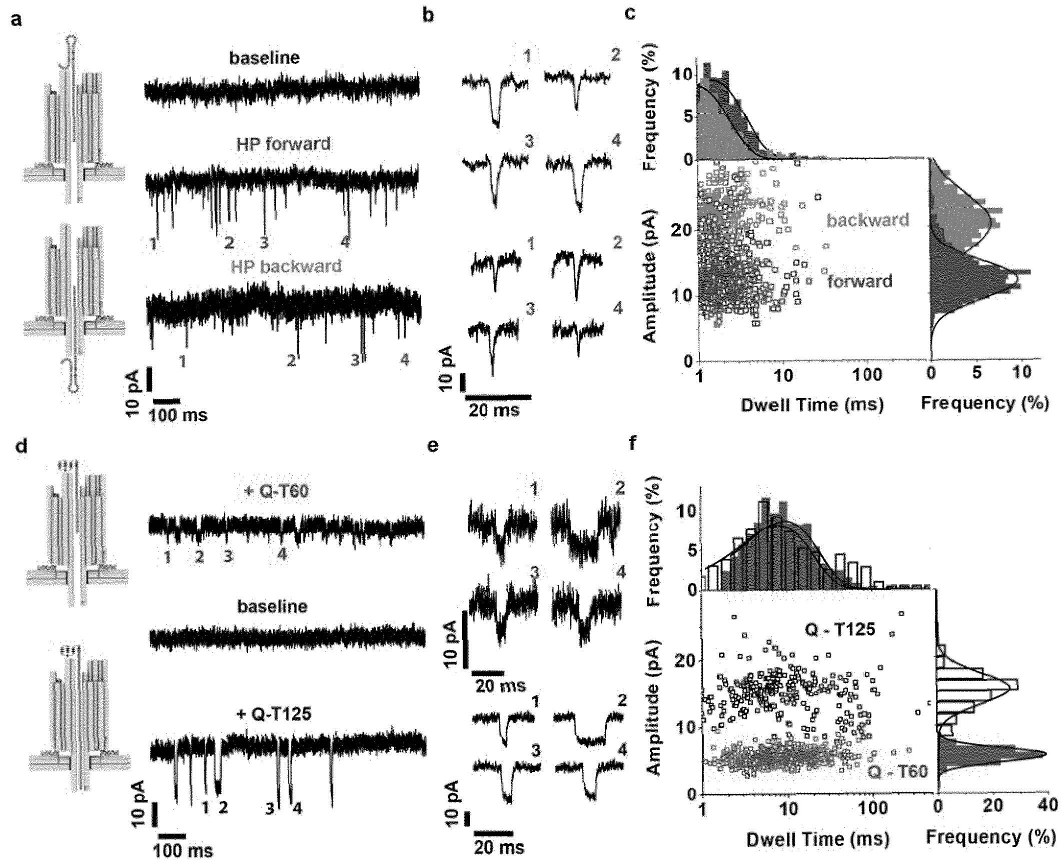
a
b
c
baseline
HP forward
HP backward
10 pA
100 ms
20 ms
Frequency (%)
Amplitude (pA)
backward
forward
Dwell Time (ms)
Frequency (%)
d
e
f
+ Q-T60
baseline
+ Q-T125
Q - T125
Q - T60

**Figure 4**

A

B

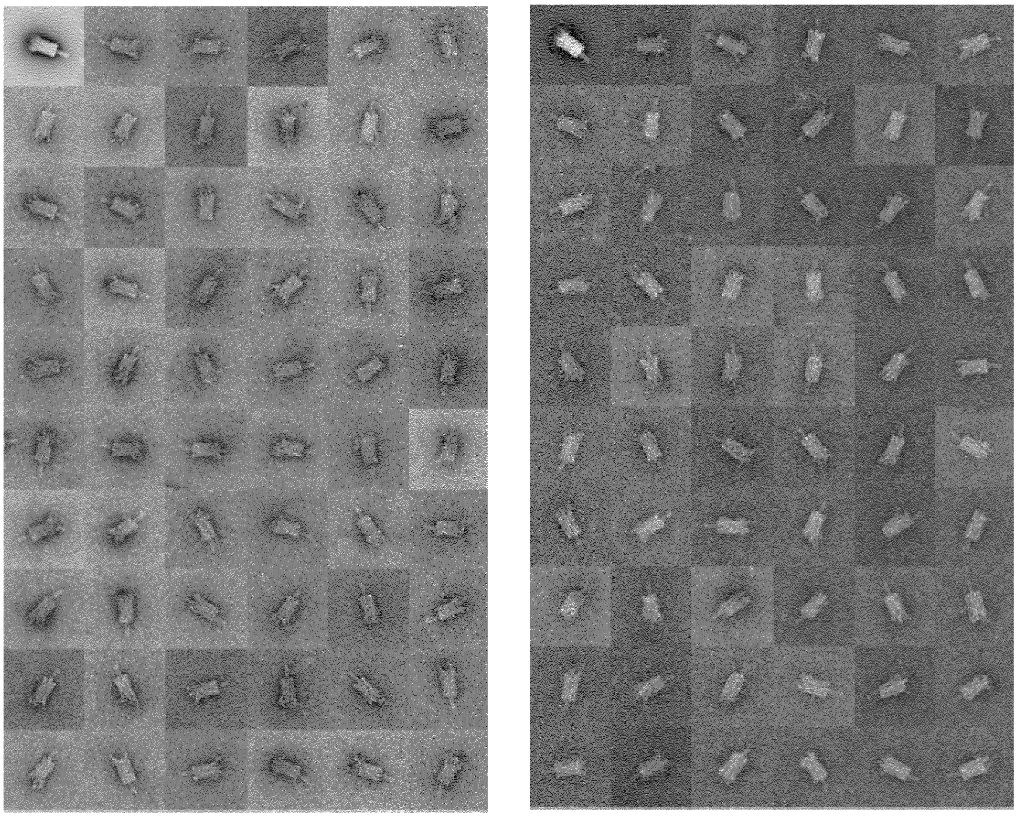

# Figure 4 C

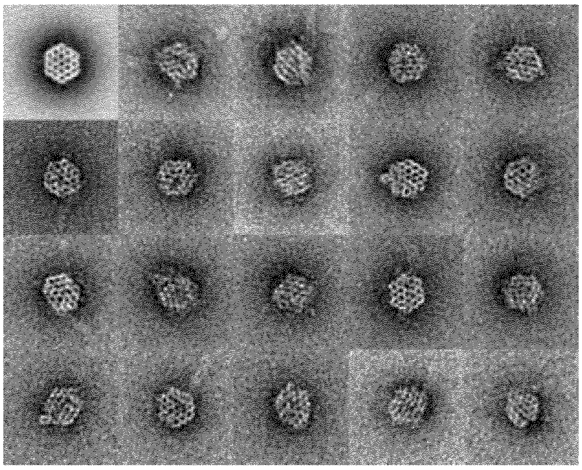

Figure 5

A

B

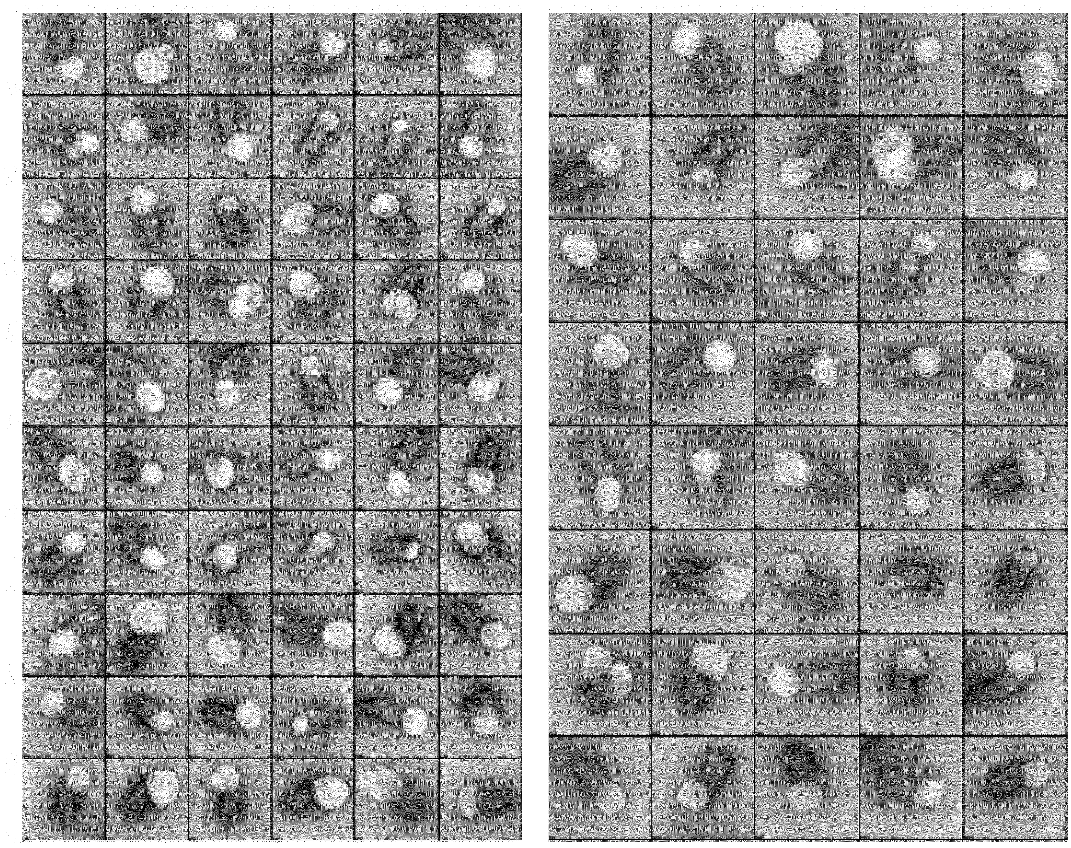

Figure 6

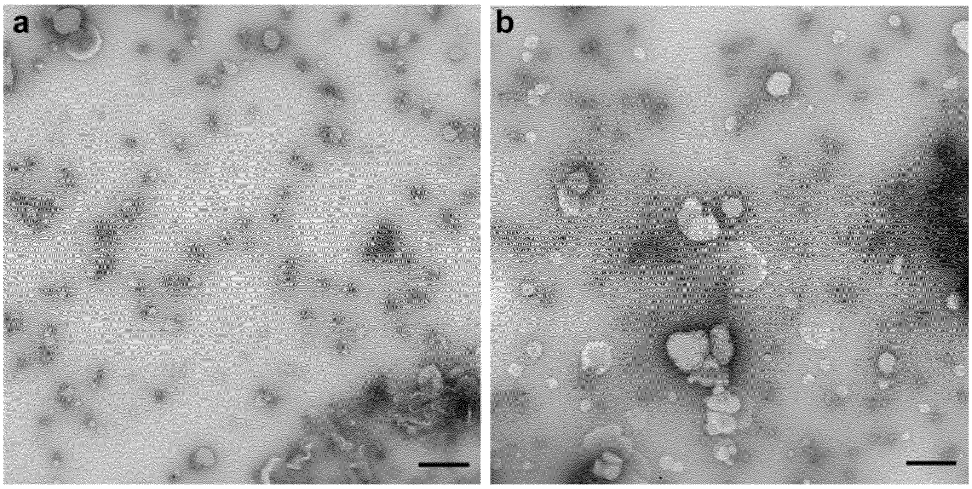


Figure 7

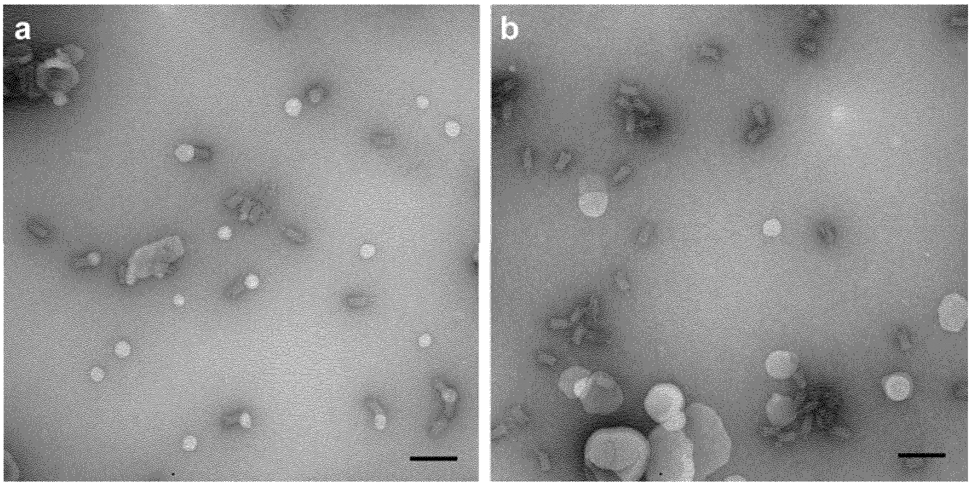

# Figure 8

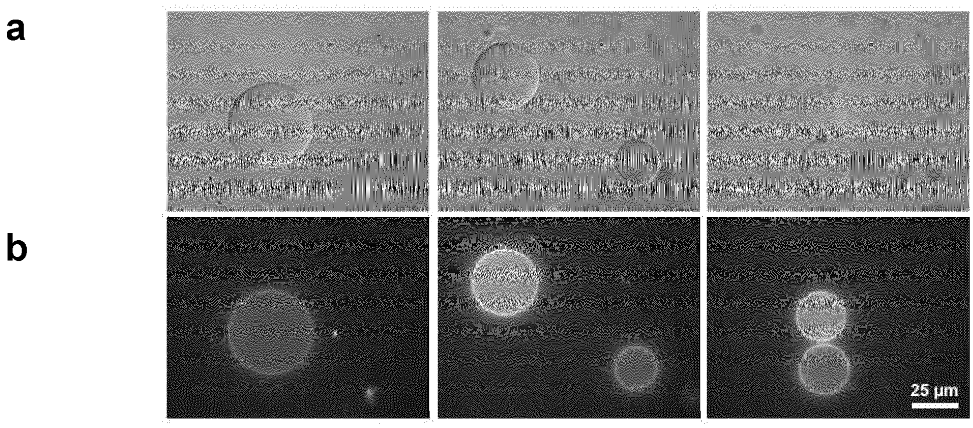


# Figure 9

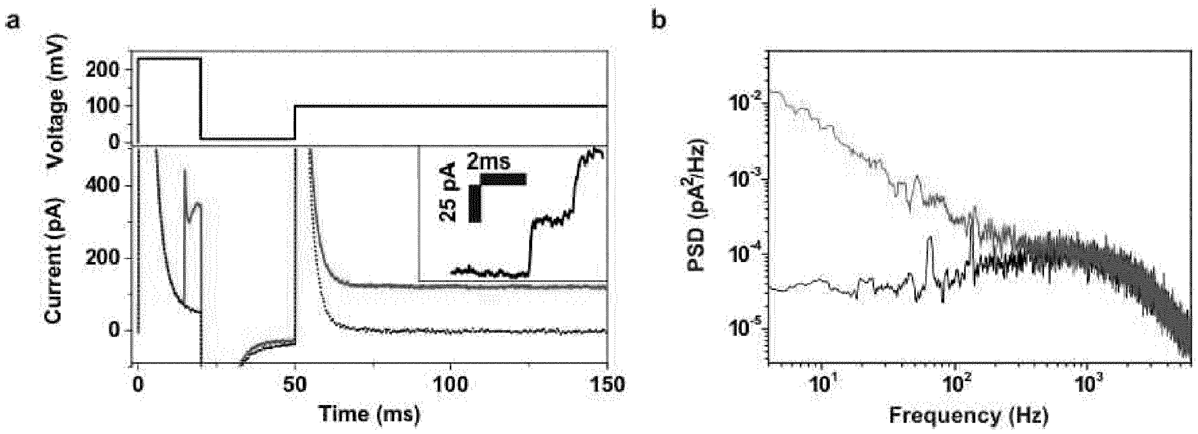

# Figure 10

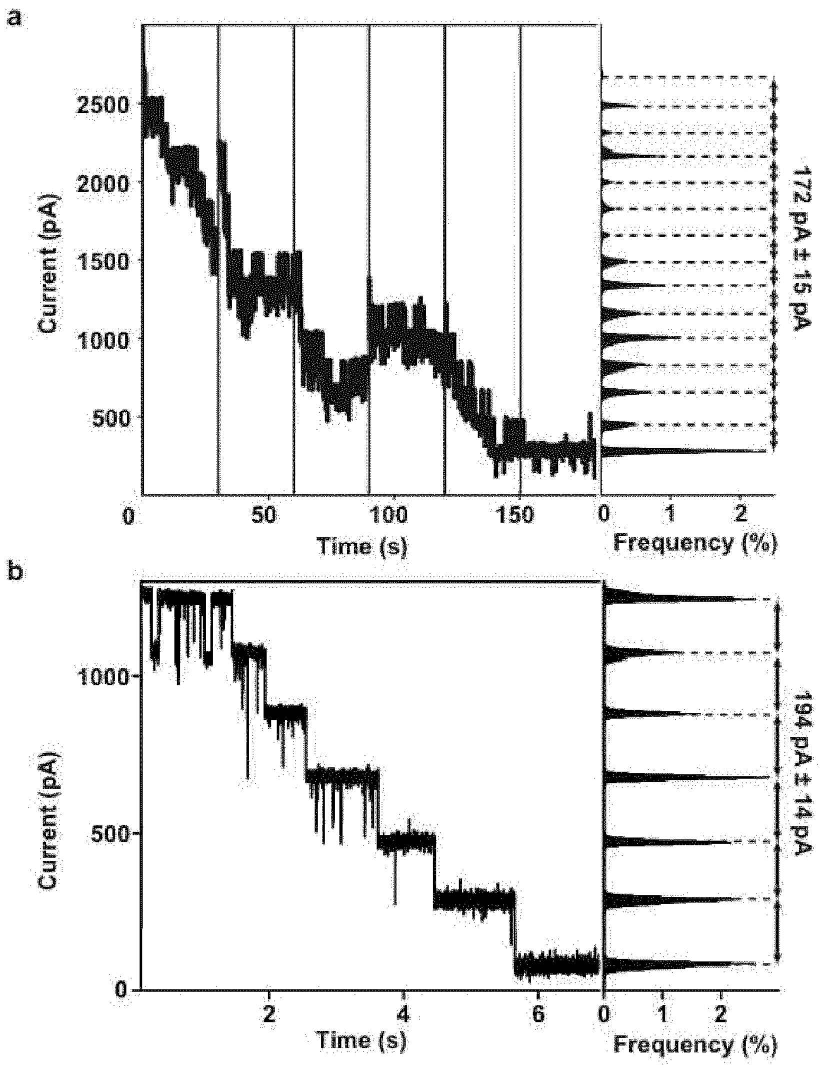
a
2500
2000
1500
1000
500
Current (pA)
0
50
100
150
Time (s)
0
1
2
Frequency (%)
172 pA ± 15 pA
b
1000
500
0
Current (pA)
2
4
6
Time (s)
0
1
2
Frequency (%)
194 pA ± 14 pA

# Figure 11

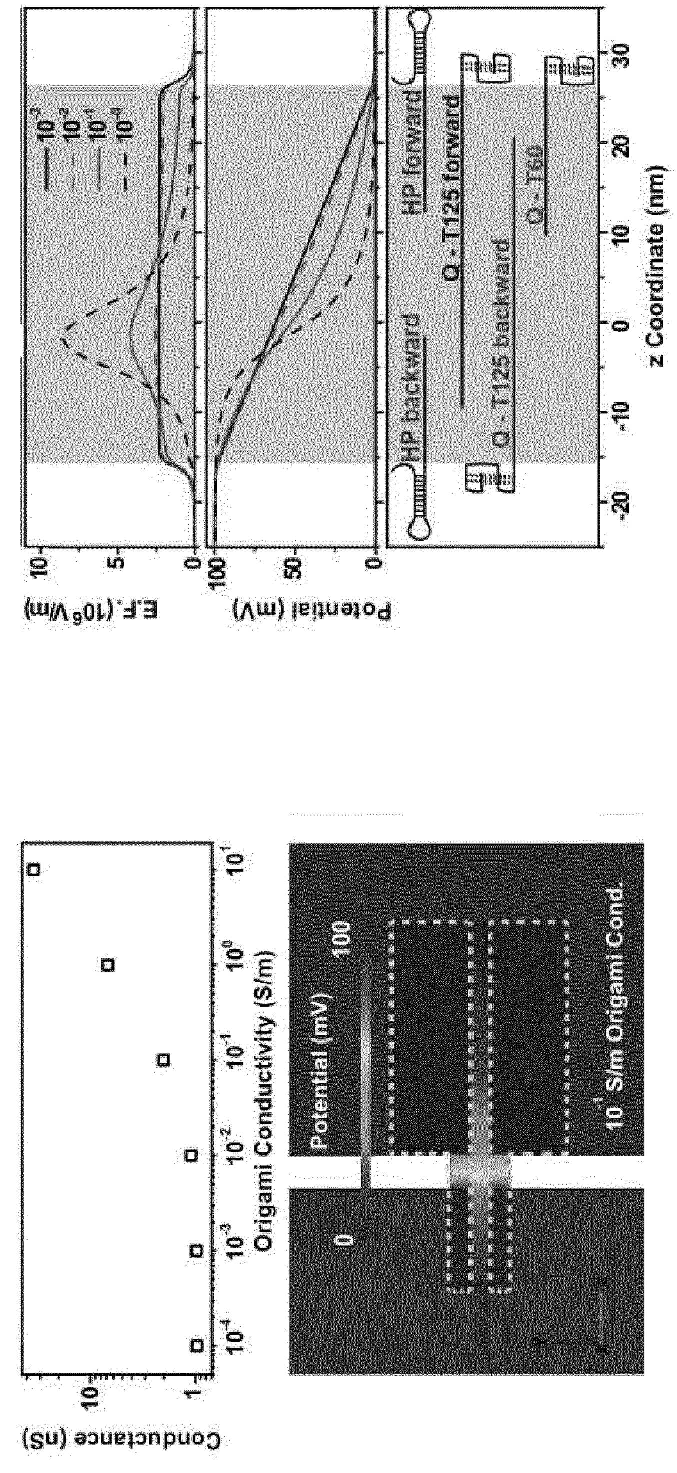

E.F. ($10^6$ V/m)
Potential (mV)
z Coordinate (nm)
$10^{-3}$
$10^{-2}$
$10^{-1}$
$10^{0}$
HP backward
HP forward
Q - T125 forward
Q - T125 backward
Q - T60
Conductance (nS)
Origami Conductivity (S/m)
Potential (mV)
0
100
$10^{-1}$ S/m Origami Cond.

# Figure 12

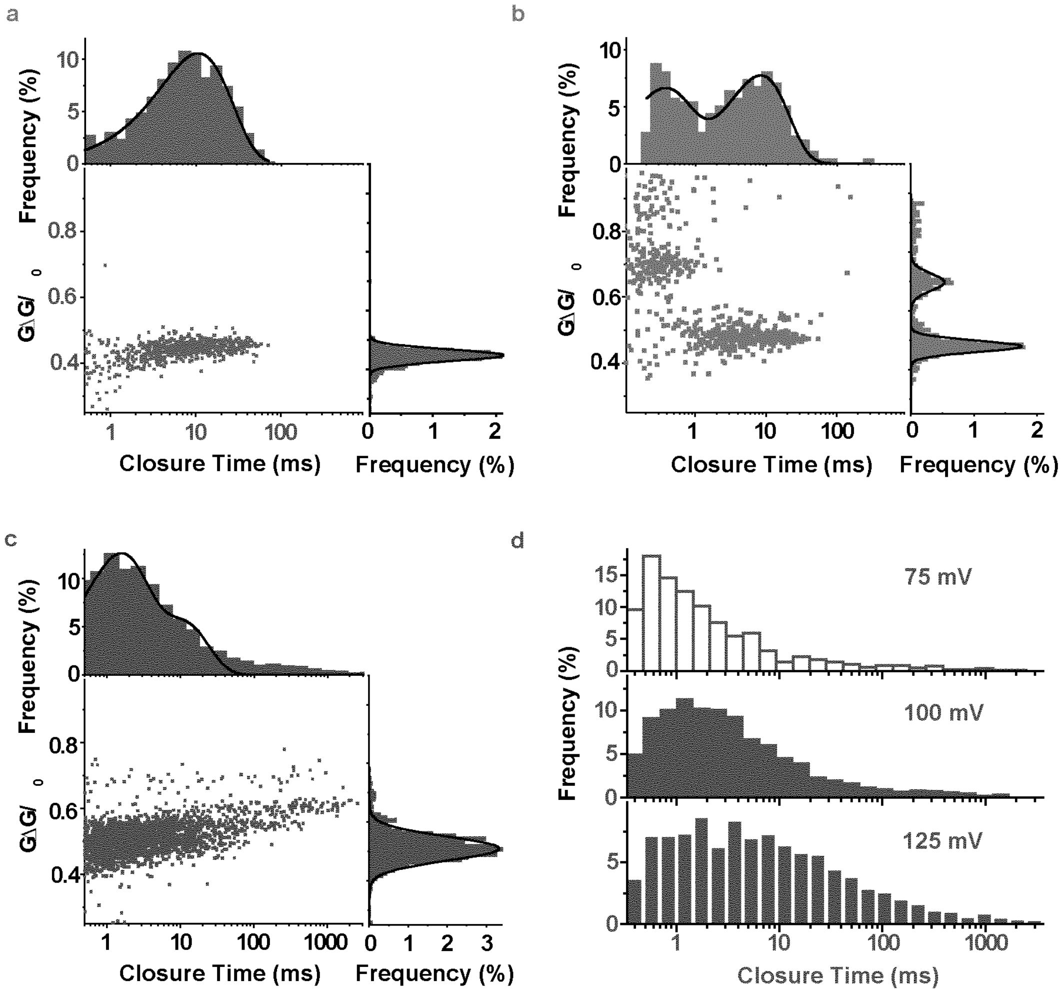
a
b
c
d
Frequency (%)
G\G/0
Closure Time (ms)
Frequency (%)
75 mV
100 mV
125 mV
Closure Time (ms)

# Figure 13

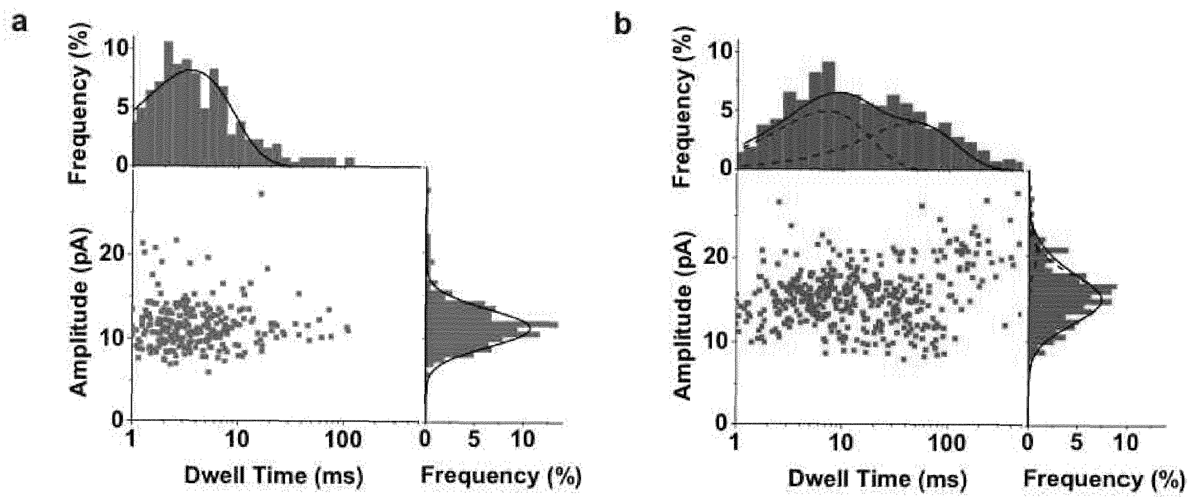
a
Frequency (%)
10
5
0
Amplitude (pA)
20
10
0
1
10
100
Dwell Time (ms)
0
5
10
Frequency (%)
b
Frequency (%)
10
5
0
Amplitude (pA)
20
10
0
1
10
100
Dwell Time (ms)
0
5
10
Frequency (%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7842793 B2, Rothemund


### Non-patent literature cited in the description


- **ANDERSEN, E. S. et al.** Self-assembly of a nanoscale DNA box with a controllable lid. *Nature,* 2009, vol. 459, 73-76
- **AKHSHI P ; MOSEY NJ ; WU G.** Free-energy landscapes of ion movement through a G-quadruplex DNA channel. *Angew Chem Int Ed Engl.,* 2012, vol. 51 (12), 2850-2854
- **AKSIMENTIEV, A. ; SCHULTEN, K.** Imaging a-Hemolysin with Molecular Dynamics: Ionic Conductance, Osmotic Permeability, and the Electrostatic Potential Map. *Biophys,* 2008, vol. J88, 3745-3761
- **BAAKEN, G. ; SONDERMANN, M. ; SCHLEMMER, C. ; RUEHE, J. ; BEHRENDS, J. C.** Planar microelectrode-cavity array for high-resolution and parallel electrical recording of membrane ionic currents. *Lab Chip,* 2008, vol. 8, 938-944
- **BELL, N. A. W. et al.** DNA Origami Nanopores. *Nano Lett,* 2012, vol. 12, 512-517
- **BOYER, P. D.** *Annu. Rev. Biochem.,* 1997, vol. 66, 717-749
- **BRANTON, D. et al.** The potential and challenges of nanopore sequencing. *Nat. Biotech.,* 2008, vol. 26, 1146-1153
- **BURGE, S. ; PARKINSON, G. N. ; HAZEL, P. ; TODD, A. K. ; NEIDLE, S.** Quadruplex DNA: sequence, topology and structure. *Nucleic Acids Res.,* 2006
- **CASTRO, C. E. et al.** A primer to scaffolded DNA origami. *Nat Meth,* 2011, vol. 8, 221-229
- **DIETZ, H. ; DOUGLAS, S. M. ; SHIH, W. M.** Folding DNA into Twisted and Curved Nanoscale Shapes. *Science,* 2009, vol. 325, 725-730
- **DOUGLAS, S. M. et al.** Rapid prototyping of 3D DNA-origami shapes with caDNAno. *Nucleic Acids Res.,* 2009, vol. 37, 5001-5006
- **DOUGLAS, S. M. et al.** Self-assembly of DNA into nanoscale three-dimensional shapes. *Nature,* 2009, vol. 459, 414-418
- **FORMAN SL et al.** Toward Artificial Ion Channels: A Lipophilic G-Quadruplex. *J. Am. Chem. Soc.,* 2000, vol. 122, 4060-4067
- **HALL, A. R. et al.** Hybrid pore formation by directed insertion of [alpha]-haemolysin into solid-state nanopores. *Nat Nano,* 2010, vol. 5, 874-877
- **HAN, D. et al.** DNA origami with complex curvatures in three-dimensional space. *Science,* 2011, vol. 332, 342-346
- **HILLE, B.** Ion channels of excitable membranes. Sinauer Associates, 2001
- **KAUCHER MS ; HARRELL WA JR ; DAVIS JT.** A unimolecular G-quadruplex that functions as a synthetic transmembrane Na+ transporter. *JAm Chem Soc.,* 2006, vol. 128 (1), 38-39
- **MAAREL, J. R. C.** Introduction to Biopolymer Physics. World Scientific, 2007
- **MATHE, J. ; VISRAM, H. ; VIASNOFF, V. ; RABIN, Y. ; MELLER, A.** Nanopore Unzipping of Individual DNA Hairpin Molecules. *Biophys. J.,* 2004, vol. 87, 3205-3212
- **PRATT, K. ; KOCH, W. ; WU, Y. ; BEREZANSKY, P.** Molality-based primary standards of electrolytic conductivity - (IUPAC technical report). *Pure Appl. Chem.,* 2001, vol. 73, 1783-1793
- **ROTHEMUND, P. W. K.** Folding DNA to create nanoscale shapes and patterns. *Nature,* 2006, vol. 440, 297-302
- **SAKMANN, B. ; NEHER, E.** Single Channel Recording. Plenum, 1995
- **SCHERES, S. H. W.** Modeling experimental image formation for likelihood-based classification of electron microscopy. *Structure,* 2007, vol. 15, 1167-1177
- **SEEMAN, N. C.** Nanomaterials Based on DNA. *Annu. Rev. Biochem.,* 2010, vol. 79, 65-87
- **SONG, L. et al.** Structure of staphylococcal alpha-hemolysin, a heptameric transmembrane pore. *Science,* 1996, vol. 274, 1859-1866
- **TANG, G. et al.** EMAN2: An extensible image processing suite for electron microscopy. *J. Struct. Biol.,* 2007, vol. 157, 38-46
- **WEI, R. ; MARTIN, T. G. ; RANT, U. ; DIETZ, H.** DNA Origami Gatekeepers for Solid-State Nanopores. *Angewandte Chemie International Edition,* 2012, vol. 51 (20), 4864-7